# EUROPÄISCHE PATENTSCHRIFT

(11) **EP 3 419 707 B1**
(45) Veröffentlichungstag und Bekanntmachung des Hinweises auf die Patenterteilung: **25.01.2023**
(21) Anmeldenummer: 17705148.9
(22) Anmeldetag: 20.02.2017
(51) Int. Cl.: A61M 15/00

(54) **INHALATOR**
INHALER
INHALATEUR

(30) Priorität: 24.02.2016 EP 16020049
(43) Veröffentlichungstag der Anmeldung: 02.01.2019
(73) Patentinhaber: Boehringer Ingelheim International GmbH, 55216 Ingelheim am Rhein (DE)
(72) Erfinder: HOLAKOVSKY, Holger, 55216 Ingelheim am Rhein (DE); BESSELER, Jens, 55216 Ingelheim am Rhein (DE); FRENTZEL-BEYME, Jessica, 55216 Ingelheim am Rhein (DE); HERRMANN, Frank, 55216 Ingelheim am Rhein (DE)
(74) Vertreter: Simon, Elke Anna Maria
(86) Internationale Anmeldenummer: PCT/EP2017/053728
(87) Internationale Veröffentlichungsnummer: WO 2017/144386

(56) Entgegenhaltungen:
- EP-A2- 1 106 196
- WO-A1-02/24268
- WO-A1-02/36189
- WO-A1-91/02558
- WO-A1-92/00812
- WO-A1-2004/052435
- WO-A1-2006/071512
- WO-A1-2007/118648
- US-A1- 2002 170 560

## Beschreibung

Die vorliegende Erfindung betrifft einen Inhalator gemäß dem Oberbegriff des Anspruchs 1.

Die vorliegende Erfindung betrifft insbesondere einen Inhalator zur Ausgabe bzw. Inhalation einer vorzugsweise pulverförmigen Formulierung, also einen Pulverinhalator.

Bei der Formulierung handelt es sich insbesondere um ein therapeutisches Mittel bzw. Arzneimittel. Insbesondere enthält die Formulierung dementsprechend mindestens einen Wirkstoff oder besteht daraus. Die Formulierung dient also insbesondere der medizinischen Behandlung oder sonstigen therapeutischen Zwecken.

Die US 2002/170560 A1 offenbart einen Pulverinhalator, der eine Blisterscheibe beinhaltet, auf der sich mehrere Dosen eines pharmazeutischen Pulvers in Blistern befindet. Die Blisterscheibe ist auf einem Schubfach-Einsatz angeordnet, das mit der Blisterscheibe austauschbar zwischen einer Basisplatte und einer oberen Abdeckung eingeschoben wird. An der Basisplatte ist ein Mundstück angebracht, das mit einer Staubkappe verschlossen werden kann. Der Inhalator weist einen pivotal bewegbaren Aktuator auf, bei dessen Betätigung in eine erste Richtung ein Blister im Innern des Inhalators geöffnet wird. Bei entgegengesetzter pivotaler Bewegung des Aktuators wird die Blisterscheibe im Schubfach zur Positionierung des nächsten Blisters vorgerückt.

Die WO02/36189 A1 zeigt einen Medikamentenabgabegerät, das einen Körper und einen dazu relativ bewegbaren Halter aufweist, der im Innern eine Kassette mit einem Medikamententräger aufnehmen kann. Der Medikamententräger ist ein Blisterstreifen mit einer Vielzahl von diskreten Dosen eines pulverförmigen Medikaments und das Gerät weist einen Mechanismus auf, um die Dosen zur Inhalation zugänglich zu machen. Eine Bewegung des Halters relativ zum Körper resultiert in einer Bewegung der Kassette zwischen einer ersten und einer zweiten Position, wobei die Kassette in der zweiten Position reversibel aus dem Halter entfernt werden kann. Die erste Position ist die Medikamenten-Abgabe Position der Kassette.

Die WO 92/00812 A1 zeigt eine Austragsvorrichtung, mit der durch Drehschaltung aufeinanderfolgend dosierte Medienmengen aus gesonderten, in einem Kranz angeordneten Zylinderbehältern entnommen werden können. Jeder Zylinderbehälter bildet zweckmäßig mit einem eingesetzten Pumpkolben und einem Verschluss eine vorgefüllte Baueinheit, deren Pumpkolben bei der Betätigung der Austragsvorrichtung mit einem Stößel gekoppelt wird. Die Austragsvorrichtung ist an ihrer Außenseite im Wesentlichen von zwei Gehäusekörpern begrenzt, die nach Art einer flachen Dose mit Deckel ineinandergreifen und im Innern die Vielzahl von Pumpen aufnehmen. Durch eine magazinartige Ausbildung eines der Gehäuseteile lässt sich dieses Magazin nach Trennung der beiden Gehäuseteile wieder mit frischgefüllten Pumpeneinheiten nachrüsten.

Bei der vorliegenden Erfindung ist die Formulierung in Kapseln aufgenommen, wobei jede Kapsel eine Dosis der Formulierung enthält. Die Formulierung ist also in die Kapseln vordosiert. Bei der vorliegenden Erfindung sind unter dem Begriff "Kapsel" primär Behältnisse mit einer festen oder einer zumindest im wesentlichen starren, insbesondere dichten, einstückigen, geschlossenen und/oder durchgängigen Hülle zu verstehen, die insbesondere separat voneinander handhabbar und/oder öffenbar sind. Vorzugsweise sind in einem weiteren Sinn gemäß der vorliegenden Erfindung unter dem Begriff "Kapsel" auch sonstige Behältnisse, Verpackungen oder dergleichen mit jeweils einer Dosis der Formulierung zu verstehen, die insbesondere separat voneinander handhabbar und/oder öffenbar sind.

Die EP 0 147 755 A2 und WO 2004/052435 A1 offenbaren Inhalatoren für die Inhalation pulverförmiger Arzneimittel aus länglichen Kapseln. Der Inhalator weist jeweils eine Kapselkammer auf, in die jeweils eine Kapsel manuell einführbar ist. Die Kapsel wird durch manuelle Betätigung einer Öffnungseinrichtung in der Kapselkammer längsseitig angestochen und dadurch geöffnet. Beim Inhalieren führt ein durch die Kapselkammer strömender Luftstrom dazu, dass die Kapsel in der Kapselkammer hin- und her bewegt wird, wobei das pulverförmige Arzneimittel ausgetragen und im Luftstrom dispergiert wird. Die vorliegende Erfindung benutzt insbesondere dieses Prinzip, ist jedoch aber auch bei sonstigen Techniken zum Austrag einer Formulierung einsetzbar.

Die WO 2005/049121 A1 offenbart einen Pulverinhalator mit einer Vielzahl von Kapseln. Die zylindrischen Kapseln sind aufrecht hintereinander von einer Schiene geführt oder kettenartig miteinander verbunden.

Die WO 2007/118648 A1 offenbart einen Pulverinhalator, der insbesondere mehrere Kapselkammern mit darin aufgenommenen Kapseln aufweist, wobei jede Kapselkammer insbesondere nur einmalig verwendet wird. Die Kapseln und Kapselkammern können radial ausgerichtet sein, wobei die Auslassöffnungen der Kapselkammern von einer gemeinsamen Abdeckung abgedeckt sein können. Dabei können die Kapseln und Kapselkammern auch in zwei axial versetzten Ebenen angeordnet sein. Gemäß einer anderen Ausführungsform kann der Inhalator auch nur eine Kapselkammer zur einzelweisen Aufnahme von Kapseln nacheinander zu deren Entleerung bei der Inhalation aufweisen. Weiter kann eine Mundstückabdeckung beim Schwenken die Kapseln vorwärts und einzelweise in die Kapselkammer bewegen.

Die WO 2011/039307 A2 zeigt einen Inhalator für die Inhalation pulverförmiger Arzneimittel aus Kapseln mit einem wechselbaren Austauschrohr, das eine Kapselkammer mit einem sich anschließenden Ausgabekanal bildet, wobei in die Kapselkammer eine Kapsel voreingesetzt ist. Das Austauschrohr weist Öffnungen für Nadeln zum Ausstechen der jeweiligen Kapsel auf, wobei die Öffnungen von einer Membran selbsttätig verschlossen werden können.

Eine Kapselkammer im Sinne der vorliegenden Erfindung ist vorzugsweise ein zumindest im Wesentlichen starres oder festes und/oder längliches Behältnis bzw. Kapselgehäuse mit einer insbesondere länglichen oder zylindrischen Kammer, in der die jeweilige Kapsel zur Entleerung insbesondere hin- und her bewegbar oder auf sonstige Weise bewegbar bzw. in Vibration oder Schwingung versetzbar ist. Vorzugsweise weist die Kapselkammer einen Einlass und einen Auslass für Luft, insbesondere an ihren entgegengesetzten Enden bzw. Stirnseiten, auf.

Der vorliegenden Erfindung liegt die Aufgabe zugrunde, einen Inhalator und ein Magazin anzugeben, der eine einfache Handhabung und/oder einen einfachen bzw. kompakten Aufbau gestatten.

Die obige Aufgabe wird durch einen Inhalator gemäß Anspruch 1 gelöst. Vorteilhafte Weiterbildungen sind Gegenstand der Unteransprüche.

Gemäß einem Aspekt der vorliegenden Erfindung weist der Inhalator bzw. dessen Gehäuse vorzugsweise ein einsetzbares oder wechselbares Gehäuseteil auf, das unlösbar mit einem drehbaren Magazin gekoppelt ist, das seinerseits Kapseln mit einer zu inhalierenden Formulierung enthält. Dies gestattet ein sehr einfaches, sicheres und/oder intuitives Einsetzen bzw. Wechseln des Magazins.

Bevorzugt hat der Inhalator bzw. dessen Gehäuse eine flache und/oder im Wesentlichen scheiben- oder diskusförmige Form, wobei das vorzugsweise ringförmige Magazin seitlich bzw. in radialer Richtung in den Inhalator bzw. das Gehäuse eingesetzt wird. Der Inhalator bzw. dessen Gehäuse ohne das wechselbare Gehäuseteil weist vorzugsweise eine seitlich angeordnete Einführöffnung für das Magazin auf ("seitlich" bezieht sich hier auf den Randbereich bzw. die Randbereiche des Geräts, die quer zur Hauptebene des Geräts liegen). Insbesondere verschließt das mit dem Magazin gekoppelte Gehäuseteil die Einführöffnung (wenn das Magazin im Inhalator eingesetzt ist). Durch die seitliche Anordnung der Einführöffnung bzw. durch das seitliche Einschieben des Magazins kann der Querschnitt der Einführöffnung so klein wie möglich gehalten werden, so dass das Innere des Inhalators so wenig wie möglich Verschmutzung von außen ausgesetzt ist. Bevorzugt wird das Magazin in einer geradlinigen Bewegung in den Inhalator eingesetzt.

Gemäß einem anderen Aspekt der vorliegenden Erfindung ist das Magazin vorzugsweise durch Öffnen und/oder Schließen einer Abdeckung, die einem Mundstück des Inhalators zugeordnet ist schrittweise bewegbar bzw. drehbar, wobei die Bewegungsschritte insbesondere derart ausgebildet sind, dass durch Öffnen oder Schlie-ßen der Abdeckung das Magazin in Relation zum Mundstück von einer Kapsel zur nächsten Kapsel weiter bewegt bzw. weitergedreht wird. Bedarfsweise kann der Antrieb auch indirekt dadurch erfolgen, dass die Abdeckung ein Speicher- oder Federelement antreibt oder spannt, das seinerseits zu einem geeigneten Zeitpunkt die gewünschte Bewegung bewirkt.

Alternativ oder zusätzlich ist es auch möglich, dass durch Betätigung eines Betätigungselements, wie eines Knopfs oder dergleichen, das Magazin zur nächsten Kapsel weiterbewegbar bzw. weiterdrehbar ist. Beispielsweise kann ein Betätigung des Betätigungselements die Weiterbewegung auch nur freigegeben werden, so dass das Magazin durch die Kraft oder Energie eines Federspeichers oder sonstigen Energiespeichers nach entsprechender Freigabe, insbesondere nur bis zur nächsten Kapsel oder Kapselkammer, weiterbewegt bzw. weitergedreht wird. Ein weiterer Aspekt der vorliegenden Erfindung liegt darin, dass der Inhalator vorzugsweise ein von außen an die Kapselkammer anlegbares Dichtelement zum Verschließen einer Nadelöffnung bei der Inhalation aufweist. Dies gestattet einen einfachen Aufbau und eine sichere Funktion.

Gemäß einem anderen Aspekt der vorliegenden Erfindung sind die Kapselkammern vorzugsweise als vorgefertigte Teile in das Magazin eingesetzt. Dies gestattet einen einfachen bzw. optimierten Aufbau, wobei beispielsweise für die Kapseln einerseits und einem Träger des Magazins zum Halten der Kapseln andererseits unterschiedliche Materialien eingesetzt werden können.

Gemäß einem anderen Aspekt der vorliegenden Erfindung sind die Kapseln bzw. Kapselkammern vorzugsweise mittels eines gemeinsamen insbesondere ringförmigen Sicherungselements am bzw. im Magazin derart gesichert, dass sie in den Kapselkammern bzw. im Magazin verbleiben und gleichzeitig Öffnungen, insbesondere Auslassöffnungen vorliegen, so dass eine Abgabe der Formulierung aus der jeweiligen Kapsel nach Öffnen der Kapsel noch möglich ist. Insbesondere ist das Sicherungselement drahtförmig und/oder als Spannring ausgebildet. Vorzugsweise ist das Sicherungselement am äußeren Umfang des Magazins angeordnet. Dies gestattet einen einfachen Aufbau, insbesondere hinsichtlich der Befüllung der Kapselkammern mit Kapseln und deren Rückhaltung in den Kapselkammern.

Nach Entfernen des Sicherungselements könnte das Magazin nach seiner Verwertung geleert, d.h. die gebrauchten Kapseln entnommen, und mit neuen Kapsel wieder befüllt werden. Um sicherzugehen, dass keine Formulierungsreste von den vorherigen Benutzungen in den Kapselkammern anhaften, sollte das Magazin aber vor Einsatz neuer Kapseln und erneutem Anbringen eines Sicherungselements gründlich gereinigt werden. Aufgrund der Wichtigkeit, dass das Sicherungselement wirklich korrekt angebracht wird, wird das Magazin vorzugsweise nur einmalig in den Inhalator eingesetzt bzw. jede Kapselkammer nur einmalig verwendet, d.h. das Magazin und das damit verbundene Gehäuseteil bilden vorzugsweise ein Wegwerf-Teil.

Gemäß einem weiteren Aspekt der vorliegenden Erfindung sind die Kapseln vorzugsweise länglich ausgebildet und mit ihren Längsachsen schräg zur Ringebene bzw. einer Drehachse des Magazins ausgerichtet. Dies gestattet einen einfachen und/oder kompakten Aufbau und/oder eine einfache bzw. intuitive Handhabung, da eine für einen Benutzer angenehme Wölbung des Inhalators auf einer Flachseite und/oder eine geneigte Anordnung von Bedienelementen, wie eine Anstechvorrichtung, ermöglicht wird.

Die vorgenannten Aspekte sowie die sich aus der folgenden Beschreibung ergebenden Aspekte der vorliegenden Erfindung können unabhängig voneinander, aber auch in beliebiger Kombination registriert werden.

Einzelne Aspekte, Merkmale, Eigenschaften und Vorteile der vorliegenden Erfindung ergeben sich aus den Ansprüchen und der folgenden Beschreibung bevorzugter Ausführungsformen anhand der Zeichnung. Es zeigt:
- Fig. 1: eine schematische Schnittdarstellung eines vorschlagsgemäßen Inhalators zur Veranschaulichung des Funktionsprinzips;
- Fig. 2: eine Draufsicht eines Inhalators gemäß einer ersten vorschlagsgemäßen Ausführungsform;
- Fig. 3: eine explosionsartige Ansicht des Inhalators gemäß Fig. 2 mit separatem Magazin und Gehäuseteil;
- Fig. 4: eine schematische Darstellung des Einsetzens des Magazins des Gehäuseteils in den Inhalator;
- Fig. 5: eine perspektivische Ansicht des vorschlagsgemäßen Magazins;
- Fig. 6: einen ausschnittsweisen Schnitt des Magazins und Gehäuseteils in radialer Richtung;
- Fig. 7: eine explosionsartige Darstellung des Magazins;
- Fig. 8: eine perspektivische Ansicht einer Kapselkammer des Magazins;
- Fig. 9: einen schematischen Schnitt des Inhalators im Bereich eines Betätigungselements mit einer Einrichtung zum Antrieb des Magazins;
- Fig. 10: eine perspektivische Ansicht eines vorschlagsgemäßen Magazins gemäß einer zweiten Ausführungsform;
- Fig. 11: eine Seitenansicht eines vorschlagsgemäßen Inhalators gemäß einer zweiten Ausführungsform;
- Fig. 12: einen schematischen Schnitt des Inhalators gemäß Fig. 11;
- Fig. 13: eine perspektivische Ansicht eines vorschlagsgemäßen Magazins gemäß einer dritten Ausführungsform;
- Fig. 14: eine perspektivische Ansicht eines vorschlagsgemäßen Inhalators gemäß einer dritten Ausführungsform;
- Fig. 15: eine andere schematische Ansicht des Inhalators gemäß Fig. 14 im Bereich eines Mundstücks und der Öffnungseinrichtung;
- Fig. 16: eine schematische Darstellung des Inhalators gemäß Fig. 14 bei geöffneter Abdeckung;
- Fig. 17: eine perspektivische Ansicht eines vorschlagsgemäßen Inhalators gemäß einer vierten Ausführungsform;
- Fig. 18: einen schematischen Vertikalschnitt des Inhalators gemäß Fig. 17 in einer Ausgangsposition;
- Fig. 19: einen schematischen Vertikalschnitt des Inhalators gemäß Fig. 17 bei betätigter Anstecheinrichtung;
- Fig. 20: einen schematischen Vertikalschnitt des Inhalators gemäß Fig. 17 nach Betätigung der Anstecheinrichtung; und
- Fig. 21: einen schematischen Vertikalschnitt des Inhalators gemäß Fig. 17 bei angehobenem bzw. abgerücktem Mundstück.

In den Figuren werden für gleiche oder ähnliche Teile die gleichen Bezugszeichen verwendet, auch wenn eine wiederholte Beschreibung weggelassen ist. Insbesondere ergeben sich auch die gleichen oder entsprechende Vorteile und Eigenschaften. Einzelne Figuren können aus Darstellungs- oder Vereinfachungsgründen auch nicht maßstabsgerecht sein.

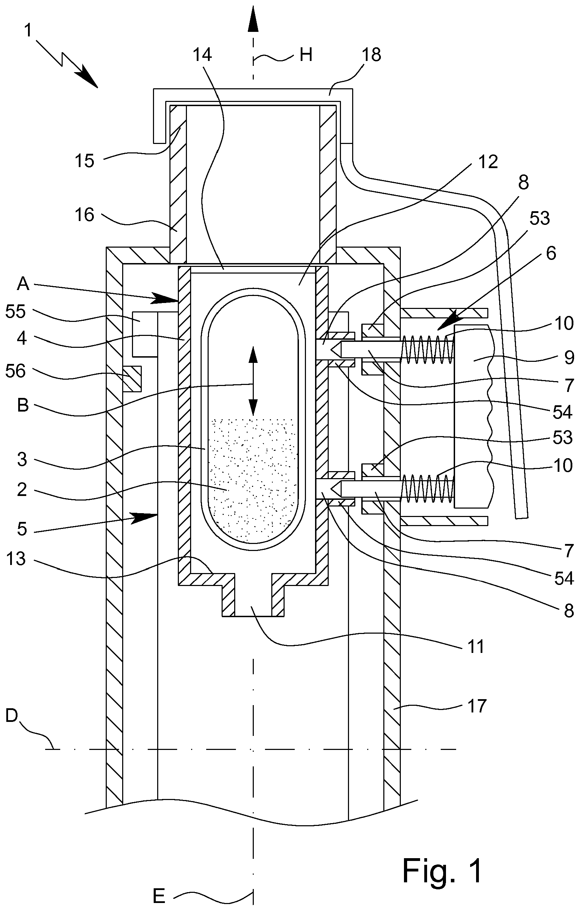

Fig. 1 zeigt in einem schematischen Schnitt den grundsätzlichen Aufbau bzw. das grundsätzliche Funktionsprinzip eines vorschlagsgemäßen Inhalators 1. Die diesbezüglichen Ausführungen gelten insbesondere entsprechend und ergänzend für alle später noch beschriebenen Ausführungsformen.

Der Inhalator 1 ist vorzugsweise tragbar ausgebildet und arbeitet insbesondere nur mechanisch.

Der Inhalator 1 dient der Ausgabe bzw. Zerstäubung, insbesondere zur Inhalation einer vorzugsweise pulverförmigen Formulierung 2 aus Kapseln 3. Die Formulierung 2 ist also vordosiert in Dosen, die in den Kapseln 3 aufgenommen sind. Bedarfsweise können die Kapseln 3 auch unterschiedliche Formulierungen 2 enthalten.

Bei der Formulierung 2 handelt es sich insbesondere um eine Formulierung im eingangs genannten Sinne.

Bei den Kapseln 3 handelt es sich insbesondere um Kapseln im eingangs genannten Sinne.

In dem schematischen Schnitt ist eine Kapsel 3 innerhalb einer Kapselkammer 4 des Inhalators 1 gezeigt. Die Kapsel 3 ist noch verschlossen, also noch nicht geöffnet.

Bei der Kapselkammer 4 handelt es sich insbesondere um eine Kapselkammer im eingangs genannten Sinne.

Die Kapseln 3 sind vorzugsweise länglich ausgebildet. Grundsätzlich können die Kapseln 3 jedoch auch jede sonstige geeignete Form aufweisen und beispielsweise kugelförmig ausgebildet sein.

Die Kapseln 3 können grundsätzlich aus jedem geeigneten Material hergestellt sein bzw. bestehen. Vorzugsweise wird Gelatine als Kapselmaterial verwendet. In diesem Fall kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird Gelatine im Gemisch mit PEG, bevorzugt PEG 3350, verwendet. Besonders bevorzugt enthält eine GelatineKapsel 3 PEG in einem Anteil von 1 bis 10 % (Gew.-%), bevorzugt 3 bis 8 %. Besonders bevorzugte Gelatine-Kapseln 3 enthalten PEG in einem Anteil von 4 bis 6 %, wobei ein PEG-Anteil von etwa 5 % höchstbevorzugt ist. Im Falle Gelatine-haltiger Kapselmaterialien weisen die Kapseln 3 vorzugsweise eine Tews- oder Halogentrockner-Feuchte von weniger als 12 %, besonders bevorzugt von ≤ 10 % auf.

Werden Cellulosederivate als Kapselmaterial verwendet, so ist die Verwendung von Hydropropylmethylcellulose, Hydroxypropylcellulose, Methylcellulose, Hydroxymethylcellulose und Hydroxyethylcellulose bevorzugt. Besonders bevorzugt wird in diesem Fall Hydropropylmethylcellulose (HPMC), besonders bevorzugt HPMC 2910 als Kapselmaterial eingesetzt. Im Falle der Verwendung von Cellulosederivaten als Kapselmaterialien liegt der Grad der Tews- oder Halogentrockner-Feuchte vorzugsweise bei weniger als 8 %, besonders bevorzugt bei weniger als 5 %. Höchstbevorzugt werden Inhalationskapseln 3 aus Cellulosederivaten vor Befüllung mit einem Tiotropium-haltigen Inhalationspulver auf eine Tews- oder Halogentrockner-Feuchte von weniger als 4 %, besonders bevorzugt weniger als 2 %, getrocknet.

Die Kapseln 3 können jeweils aus einem Kapselkörper und einer Kapselkappe bestehen, wie insbesondere in der WO 00/07572 A2 offenbart. Daher wird hiermit explizit auf den Inhalt der WO 00/07572 A2 in vollem Umfang Bezug genommen. Bei diesem zweiteiligen Aufbau wird insbesondere Kunststoff als Kapselmaterial eingesetzt. Insbesondere bestehen der Kapselkörper und die Kapselkappe aus dem gleichen Material. Sie werden so miteinander verbunden, daß ein stabiler, abgeschlossener Hohlraum von definiertem Volumen gebildet wird. Besonders bevorzugt wird hierbei Kunststoff, insbesondere Polyethylen, eingesetzt. Die Kapsel 3 kann Rastelemente aufweisen, die die Kapselkappe fest mit dem Kapselkörper verbinden.

Die Kapseln 3 und ggf. die Kapselkammern 4 (sofern mehrere Kapselkammern 4 vorhanden sind) sind vorzugsweise von einem Magazin 5 des Inhalators 1 aufgenommen. In dem schematischen Schnitt gemäß Fig. 1 ist das Magazin 5 nur schematisch angedeutet.

Das Magazin 5 ist insbesondere flach, scheibenartig oder ringartig ausgebildet. Es weist insbesondere eine Mittel- oder Hauptebene E auf.

Das Magazin 5 ist vorzugsweise in den Inhalator 1 einsetzbar und/oder auswechselbar.

Die Kapseln 3 und ggf. Kapselkammern 4 sind im oder an dem Magazin 5 vorzugsweise zumindest im Wesentlichen ringförmig angeordnet bzw. aufgenommen, bevorzugt mit zumindest im Wesentlichen radialer Ausrichtung.

Das Magazin 5 ist im Inhalator 1 vorzugsweise bewegbar bzw. drehbar, hier um eine zur Ebene E vorzugsweise senkrechte Drehachse D, insbesondere so dass das Magazin 5 von einer Kapsel 3 zur nächsten Kapsel 3 bewegbar bzw. förderbar bzw. (weiter) drehbar ist, besonders bevorzugt um die Kapseln 3 einzelweise in eine Ausgabeposition A, wie beispielhaft in Fig. 1 dargestellt, zu bringen.

Wie bereits erwähnt, enthält jede Kapsel 3 vorzugsweise eine Dosis der Formulierung 2. Da das Magazin 5 eine Vielzahl von Kapseln 3 und eine entsprechende Anzahl von Dosen enthält, kann der Inhalator 1 beispielsweise für eine Woche oder mehrere Wochen oder sogar für einen Monat die Versorgung eines Benutzers bzw. Patienten mit der Formulierung 2, also einem Medikament oder dergleichen, sicherstellen.

Die Kapseln 3 sind vorzugsweise einzelweise im Inhalator 1 öffenbar. Zum Öffnen weist der Inhalator 1 eine Öffnungseinrichtung 6 auf, die der Kapselkammer 4 bzw. Kapsel 3 bzw. Ausgabeposition A zugeordnet ist. Bedarfsweise können mehrere Öffnungseinrichtungen 3 vorgesehen sein, die jeweils einer Kapselkammer 4 bzw. Kapsel 3 zugeordnet sind. Vorzugsweise ist jedoch nur eine gemeinsame Öffnungseinrichtung 6 vorgesehen.

Vorzugsweise erfolgt das Öffnen der Kapsel 3 jeweils durch Anstechen. Hierzu weist die Öffnungseinrichtung 6 vorzugsweise mindestens ein Anstechelement, wie eine Nadel 7, beim Darstellungsbeispiel zwei Nadeln 7, auf.

Der Inhalator 1 bzw. dessen Gehäuse 17 kann optional eine Nadelführung 53 aufweisen, wie in Fig. 1 angedeutet. Alternativ oder zusätzlich kann auch die Kapselkammer 4 Nadelführungen 54 aufweisen. Die Führungen 53, 54 können insbesondere rohr- oder stutzenförmig ausgebildet sein, um die Anstechelemente bzw. Nadeln 7 längsverschieblich zu führen. Jedoch sind auch andere konstruktive Lösungen möglich.

Die Kapselkammer 4 weist vorzugsweise entsprechende Anstechöffnungen, hier Nadelöffnungen 8, für die Anstechelemente bzw. Nadeln 7 auf. Vorzugsweise sind die Anstechelemente in die Anstechöffnungen vorgerückt und verschließen diese. Diese Stellung nimmt die Öffnungseinrichtung 6 bevorzugt nach dem Öffnen einer Kapsel 3 während der Inhalation ein, um die Anstechöffnungen zumindest weitestgehend abzudichten. Jedoch sind auch andere Arten des Verschlusses bzw. der Abdichtung möglich, insbesondere von innen und/oder von außen bzw. durch sich selbsttätig oder automatisch schließende Anstechöffnungen oder Verschlusseinrichtungen. Zur Abdichtung bzw. zum Verschluss können beispielsweise selbstdichtende Membranen oder zusätzliche Dichtelemente oder dergleichen eingesetzt werden.

Vorzugsweise erfolgt ein seitliches Öffnen oder Anstechen der jeweiligen Kapsel 3. Hierzu ist die vorzugsweise längliche Kapsel 3 längsseitig bzw. seitlich und/oder quer zur Hauptströmungsrichtung oder Längsachse der Kapselkammer 4 anstechbar und dadurch öffenbar.

Besonders bevorzugt wird die Kapsel 3 beim Öffnen im Bereich ihrer beiden Enden und/oder seitlich angestochen.

Das Anstechen bzw. Öffnen von Kapseln 3 erfolgt vorzugsweise wie in der EP 0 147 455 A2 beschrieben, auf deren Inhalt diesbezüglich hiermit in vollem Umfang Bezug genommen wird.

Die Öffnungseinrichtung 6 weist vorzugsweise ein Betätigungselement 9 auf, das insbesondere manuell betätigbar ist. Vorzugsweise ist durch Betätigung oder Niederdrücken oder Eindrücken des Betätigungselements 9 die jeweilige Kapsel 3 anstechbar.

Beim Darstellungsbeispiel sind die Anstechelemente bzw. Nadeln 7 vorzugsweise fest mit dem Betätigungselement 9 verbunden. Jedoch sind auch andere konstruktive Lösungen möglich.

Vorzugsweise ist die Öffnungseinrichtung 6 bzw. das Betätigungselement 9 gegen die Kraft eines Rückstellelements, wie mindestens einer Feder 10, betätigbar. Mittels des Rückstellelements bzw. der Feder 10 nehmen die Anstechelemente bzw. Nadeln 7 nach dem Betätigen bzw. Loslassen des Betätigungselements 9 vorzugsweise wieder ihre zurückgezogene Position der Ausgangsstellung, wie in Fig. 1 angedeutet, ein. Jedoch sind auch andere konstruktive Lösungen möglich.

Der hier dargestellte Inhalator 1 weist mehrere Kapselkammern 4 auf, in die jeweils eine Kapsel 3 aufgenommen ist und die jeweils insbesondere nur einmal verwendet werden. Viele dargestellte Funktionen sind jedoch auf Inhalatoren mit nur einer Kapselkammer 4 anwendbar, in die die Kapseln 3 nacheinander für die Ausgabe bzw. Entleerung aufgenommen werden. Dabei folgt dann also eine Mehrfachverwendung der Kapselkammer 4.

Die Kapselkammer 4 ist wahlweise ein- oder mehrteilig ausgebildet. Dafür kann die Kapselkammer 4 auch aus unterschiedlichen Materialien und/oder durch Zwei-Komponenten-Spritzguss aufgebaut bzw. hergestellt sein.

Die Kapselkammer 4 weist vorzugsweise einen Einlass 11 und einen Auslass 12 auf, die sich insbesondere axial bzw. stirnseitig an einen insbesondere länglichen bzw. zylindrischen bzw. zentralen Bewegungs- bzw. Aufnahmebereich der Kapselkammer 4 für die Kapsel 3 anschließen.

Besonders bevorzugt ist der Einlass 11 in seinem Durchmesser gegenüber dem Aufnahmebereich für die Kapsel 3 im Durchmesser verringert, so dass vorzugsweise eine Ringschulter 13 oder dergleichen gebildet wird, die einen Anschlag oder eine Wegbegrenzung für die Kapsel 3 bildet.

Dem Auslass 12 ist vorzugsweise ein Sicherungselement 14 zugeordnet, das beispielsweise gitterartig ausgebildet ist und/oder ein Herausbewegen der Kapsel 3 aus dem Aufnahmebereich bzw. aus der Kapselkammer 4 verhindert.

Das Sicherungselement 14 kann je nach Bedarf und Konstruktion dem Magazin 5 bzw. der Kapselkammer 4 zugeordnet und/oder daran angeordnet sein, bedarfsweise aber auch von dem Inhalator 1 bzw. einem Mundstück 15 oder Verbindungsabschnitt 16 gebildet oder gehalten sein.

Vorzugsweise entsprechen die geometrischen Verhältnisse zumindest im Wesentlichen den Angaben in der EP 0 147 755 A2, die diesbezüglich hiermit als ergänzende Offenbarung eingeführt wird.

Bei der Inhalation bzw. Ausgabe strömt Luft oder sonstiges Gas durch den Einlass 11 in die Kapselkammer 4, durch diese bzw. deren Aufnahmebereich hindurch und aus dem vorzugsweise gegenüberliegenden Auslass 12 wieder hinaus. Dieser Luft- bzw. Gasstrom kann durch das Einatmen beim Inhalieren und/oder durch einen dem Inhalator 1 zugeordneten Druckerzeuger, wie eine Luftpumpe, einem Druckgasreservoir oder dergleichen erzeugt werden.

Der genannte Luftstrom durch die Kapselkammer 4 bewirkt, insbesondere durch den Bernoulli-Effekt, dass die Kapsel 3 in der Kapselkammer 4 vibriert oder schwingt bzw. sich insbesondere axial hin- und herbewegt, wie durch Pfeil B in Fig.1 angedeutet. Diese Bewegung oder Schwingung B bewirkt oder unterstützt den Austrag der Formulierung 2 aus der geöffneten bzw. angestochenen Kapsel 3 in den Luftstrom - insbesondere in Form von sehr feinen Partikeln - und ein Dispergieren der Formulierung 2 in den Luftstrom, mit dem die Formulierung 2 schließlich über den Auslass 12 und besonders bevorzugt ein sich anschließendes Mundstück 15 des Inhalators 1 an einen nicht darstellten Benutzer bzw. Patienten ausgegeben wird.

Die Hauptausgaberichtung H der dispergierten Formulierung 2 mittels des Luftstroms bzw. des Inhalators 1 ist in Fig. 1 durch einen entsprechenden Pfeil angedeutet. Die Ausgabe ist natürlich nur bei geöffneter Abdeckung 18 und nach Öffnen der jeweiligen Kapsel 3 möglich.

Das Ausgeben bzw. Dispergieren der Formulierung 2 erfolgt insbesondere wie in der EP 0 147 755 A2 beschrieben, die diesbezüglich als ergänzende Offenbarung eingeführt wird. Jedoch kann die Formulierung 2 grundsätzlich auch auf jede sonstige geeignete Weise aus der Kapsel 3 ausgetragen werden, beispielsweise durch Rotation der Kapsel 3 quer zu ihrer Längsachse oder dergleichen.

Der Inhalator 1 weist also vorzugsweise ein Mundstück 15 oder eine sonstige Einrichtung zur Ausgabe der in dem Luft- bzw. Gasstrom dispergierten Formulierung 2 auf.

Beim Darstellungsbeispiel schließt sich das Mundstück 15 insbesondere mit einem Anschlussabschnitt 16 an, die Kapselkammer 4 bzw. den Auslass 12 an.

Der Inhalator 1 weist vorzugsweise ein Gehäuse 17 auf. Hierbei handelt es sich insbesondere um ein Außengehäuse.

Vorzugsweise ist das Mundstück 15 am Gehäuse 17 insbesondere feststehend oder unlösbar angeordnet bzw. gebildet. Jedoch sind noch andere konstruktive Lösungen möglich.

Der Inhalator 1 bzw. das Gehäuse 17 ist vorzugsweise zumindest im Wesentlichen scheibenartig und/oder flach ausgebildet.

Das Mundstück 15 ist vorzugsweise peripher am Inhalator 1 bzw. Gehäuse 17 angeordnet.

Das Mundstück 15 bzw. die Hauptausgaberichtung H des Inhalators 1 verläuft vorzugsweise zumindest im Wesentlichen radial bzw. schräg zu der Drehachse D des Magazins 5.

Der Inhalator 1 weist vorzugsweise eine dem Mundstück 15 zugeordnete Abdeckung 18 zum wahlweisen Öffnen und Schließen des Mundstücks 15 auf. Bei geöffneter Abdeckung 18 ist das Mundstück 15 zur Inhalation freigegeben. Bei geschlossener Abdeckung 18, wie in Figur 1 dargestellt, ist das Mundstück 15 vorzugsweise abgedeckt. Eine Inhalation ist also nicht möglich. In der geschlossenen Position kann die Abdeckung 18 optional, wie in Fig. 1 angedeutet, die Öffnungseinrichtung 7 bzw. deren Betätigungselement 9 mit abdecken oder auf sonstige Weise blockieren, um ein (unerwünschtes) Öffnen der Kapsel 3 in diesem Zustand durch einen Benutzer auszuschließen bzw. zu verhindern.

Die Abdeckung 18 ist vorzugsweise (um eine zur Drehachse D koaxiale oder dazu parallele, beispielsweise außen am Gehäuse 17 liegende Schwenkachse) schwenkbar oder drehbar am Inhalator 1 bzw. Gehäuse 17 gehalten oder gelagert. Alternativ oder zusätzlich ist die Abdeckung 18 vorzugsweise auch linear bzw. radial bewegbar.

Nach der Entleerung einer Kapsel 3 bzw. nach der Inhalation der Formulierung 2 einer Kapsel 3, wird die nächste Kapsel 3 in die Ausgangsposition A gebracht bzw. bewegt. Dies erfolgt insbesondere durch Weiterbewegen bzw. Drehen des Magazins 5.

Wenn nur eine Kapselkammer 4 für mehrere oder alle Kapseln 3 vorgesehen ist, wird die entleerte Kapsel 3 zunächst aus der Kapselkammer 4 herausbewegt, beispielsweise durch einlassseitiges Öffnen der Kapselkammer 4. Anschließend kann die nächste Kapsel 3 in die Kapselkammer 4 bewegt bzw. eingeführt und die Kapselkammer 4 wieder geschlossen werden. Diese neue Kapsel 3 wird dann insbesondere erst kurz vor der nächsten Inhalation geöffnet bzw. angestochen.

Ein Öffnen und Schließen der Kapselkammer 4 entfällt hingegen, wenn jede Kapsel 3 in einer separaten Kapselkammer 4 aufgenommen ist, also nur die Kapselkammern 4 zusammen mit den Kapseln 3 jeweils einzelweise in die Ausgabeposition A gebracht bzw. bewegt werden müssen.

Zur Benutzung öffnet ein nicht dargestellter Benutzer bzw. Patient den Inhalator 1 bzw. die Abdeckung 18. Hierdurch wird das Mundstück 15 freigegeben. Dann erfolgt eine Betätigung der Öffnungseinrichtung 6, also ein Anstechen der in der Ausgabeposition A befindlichen Kapsel 3.

Die Ausgabeposition A bezeichnet insbesondere eine Position der Kapsel 3 benachbart zu dem Mundstück 15 bzw. dessen Verbindungsabschnitt 16.

In der Ausgabeposition A liegt die Kapsel 3 bzw. deren Kapselkammer 4 insbesondere in Verlängerung zu dem Verbindungsabschnitt 16 bzw. Mundstück 15 und/oder mit ihrer Längsachse ausgerichtet zu und/oder in Verlängerung der Hauptausgaberichtung H und/oder in radialer Richtung, insbesondere bezogen auf die Drehachse D des Magazins 5.

Nach dem Öffnen bzw. Anstechen der Kapsel 3 kann die Inhalation erfolgen. Anschließend wird der Inhalator 1 wieder geschlossen. Weiter wird dann die nächste Kapsel 3 in die Ausgabeposition A bewegt, insbesondere durch Weiterbewegen bzw. Weiterdrehen des Magazins 5 zur nächsten Kapsel 3.

Nachfolgend werden verschiedene Ausführungsformen des vorschlagsgemäßen Inhalators 1 und Magazins 5 näher erläutert, wobei die vorherigen Ausführungen und Erläuterungen, insbesondere entsprechend oder ergänzend gelten, auch wenn eine wiederholte Beschreibung weggelassen ist.

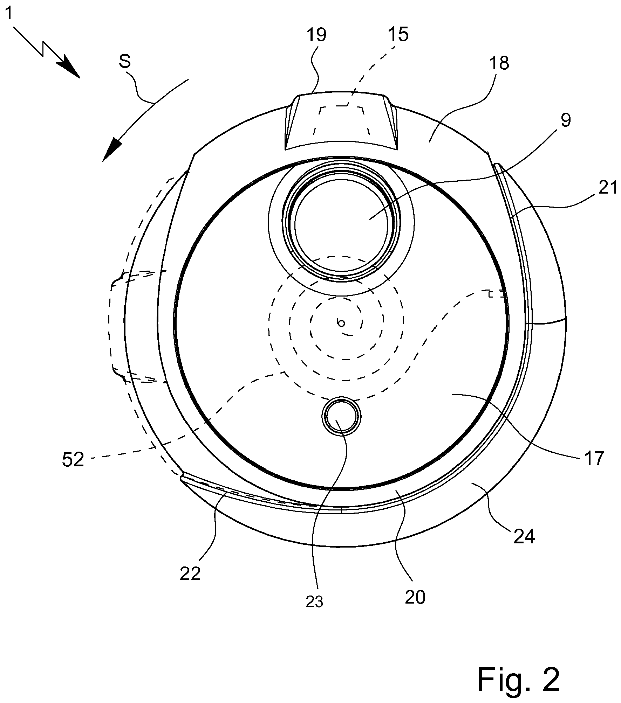

Fig. 2 zeigt in einer Draufsicht eine erste Ausführungsform des vorschlagsgemäßen Inhalators 1 bei geschlossener Abdeckung 18. Gestrichelt ist die geöffnete Position der Abdeckung 18 angedeutet.

Die Abdeckung 18 ist hier insbesondere um eine Zentralachse bzw. die Drehachse D des Magazins 5 schwenkbar. Die Schwenkachse der Abdeckung 18 ist also insbesondere koaxial oder identisch zu der Drehachse D des Magazins 5.

Beim Darstellungsbeispiel weist die Abdeckung 18 vorzugsweise eine Ausbuchtung 19 im Bereich des gestrichelt angedeuteten Mundstücks 15 zur Erleichterung einer intuitiven Erfassung der Lage des Mundstücks 15 bei geschlossener Abdeckung 18 auf.

Die Abdeckung 18 weist vorzugsweise einen ringartigen Abschnitt 20 auf einer oder beiden Seiten des Inhalators 1 bzw. Gehäuses 17 auf. Die Ringabschnitte 20 greifen jeweils vorzugsweise in entsprechende außenseitige Vertiefungen des Gehäuses 17 ein, wodurch die Abdeckung 18 in gewünschter Weise schwenkbar und unlösbar am Inhalator 1 bzw. dessen Gehäuse 17 gelagert ist. Vorzugsweise ist die Abdeckung 18 durch Aufschieben bzw. Aufrasten mit dem Inhalator 1 bzw. Gehäuse 17 verbindbar.

Beim Darstellungsbeispiel ist die Abdeckung 18 insbesondere zumindest im Wesentlichen ringförmig mit einem zum Abdecken des Mundstücks 15 verbreiterten oder vergrößerten Umfangsbereich ausgebildet.

Beim Darstellungsbeispiel deckt die Abdeckung 18 den Inhalator 1 auf der dem Betätigungselement 9 abgewandten Seite bei Bedarf auch weitgehend und/oder vollflächig ab.

Die Abdeckung 18 ist vorzugsweise von der in Fig. 2 dargestellten geschlossenen Position in die gestrichelt dargestellte geöffnete Position durch eine Schwenkbewegung S schwenkbar. In dieser geöffneten Position wird das Mundstück 15 für die Inhalation freigegeben.

Die beiden Positionen (geschlossen und geöffnet) sind vorzugsweise jeweils durch entsprechende Anschläge 21 und 22 begrenzt, die vom Inhalator 1 bzw. Gehäuse 17 oder ein Gehäuseteil 24 gebildet sind. Jedoch sind auch andere konstruktive Lösungen möglich.

Die Abdeckung 18 bzw. der Ringabschnitt 20 weist vorzugsweise zumindest auf einer Flachseite des Inhalators 1 eine zentrale Öffnung auf, so dass das Betätigungselement 9 zugänglich ist.

Beim Darstellungsbeispiel ist das Betätigungselement 9 sowohl bei geschlossener als auch bei geöffneter Abdeckung 18 zugänglich.

Beim Darstellungsbeispiel ist das Betätigungselement 9 vorzugsweise knopfförmig und/oder rund bzw. zylindrisch ausgebildet.

Der Inhalator 1 bzw. das Gehäuse 17 weist vorzugsweise eine Zähleinrichtung zum Zählen der bereits verbrauchten oder benutzten oder zum Zählen der noch zur Verfügung stehenden bzw. nicht benutzten Dosen oder Kapseln 3 auf. Die Zähleinrichtung ist beim Darstellungsbeispiel durch ein entsprechendes Fenster 23 im Gehäuse 17 und entsprechende (nicht dargestellte) Nummern am Magazin 5 bzw. an den Kapselkammern 4 gebildet. Jedoch sind auch andere konstruktive Lösungen möglich.

Der Inhalator 1 bzw. dessen Gehäuse 17 weist vorzugsweise ein Gehäuseteil 24 auf, das unlösbar mit dem drehbaren Magazin 5 gekoppelt ist und in das Gehäuse 17 einsetzbar oder daran ansetzbar ist, insbesondere um den Inhalator 1 bzw. dessen Gehäuse 17 zu schließen bzw. ein vollständiges Außengehäuse zu bilden.

Zusammen mit dem Gehäuseteil 24 ist das Magazin 5 in den Inhalator 1 einsetzbar. Je nach Gestaltung des Inhalators 1 ist es auch möglich, dass das Gehäuseteil 24 zusammen mit dem Magazin 5 gewechselt wird. Insbesondere ist das Magazin 5 nur zusammen mit dem Gehäuseteil 24 wechselbar.

Besonders bevorzugt bilden das Gehäuseteil 24 und das Magazin 5 eine Baueinheit oder Baugruppe, die von einem nicht dargestellten Benutzer oder Patienten sehr gut gegriffen und gehandhabt werden kann.

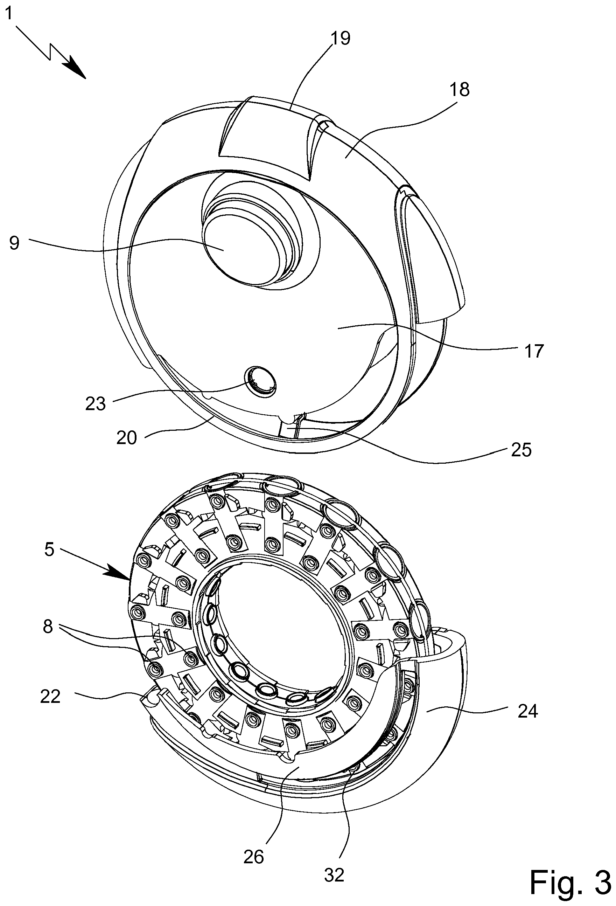

Fig. 3 zeigt in einer perspektivischen Ansicht einerseits den Inhalator 1 und andererseits das Gehäuseteil 24 mit dem zugeordneten Magazin 5 im noch nicht eingebauten Zustand, also in einem von dem Gehäuse 17 abgerückten Zustand. Die Anordnung von Gehäuse 17 (mit einer seitlichen Einführöffnung) und dem Magazin 5 in dieser Darstellung zeigt, wie das Magazin 5 vorzugsweise in geradliniger Richtung parallel zu seiner Hauptebene (bzw. in radialer Richtung im vorliegenden Ausführungsbeispiel) in das Gehäuse 17 eingeschoben werden kann. Insbesondere ist das Gehäuseteil (24), das dem Magazin 5 zugeordnet ist, derart ausgebildet, dass es nach (vollständigem) Einschub des Magazins 5 in den Inhalator 1 bzw. in das Gehäuse 17 die Einführöffnung am Gehäuse 17 verschließt. Insbesondere ist der Inhalator 1 scheiben- oder diskusförmig und das Magazin 5 ringförmig ausgebildet. Insbesondere bildet das dem Magazin 5 zugeordnete Gehäuseteil 24 einen Umfangsabschnitt entlang des Magazins 5 und/oder Gehäuses 17.

Vorzugsweise ist das Gehäuseteil 24 klemmend, rastend und/oder unlösbar mit dem Inhalator 1 bzw. Gehäuse 17 verbindbar. Gegebenenfalls kann das Verbinden bzw. Einrasten oder sichern des Gehäuseteils 24 am Inhalator 1 bzw. Gehäuse 17 auch durch das Eingreifen des Ringabschnitts 20 und/oder eines Sicherungsabschnitts 25 der Abdeckung 18, wie in Fig. 3 angedeutet, erfolgen.

Beim Darstellungsbeispiel greift der Sicherungsabschnitt 25 beispielsweise in eine sich in Umfangsrichtung erstreckende Vertiefung und/oder hinter einen entsprechenden Bogenabschnitt 26 des Gehäuseteils 24 ein, insbesondere so, dass die gewünschte Schwenkbarkeit der Abdeckung 18 ermöglicht wird oder erhalten bleibt. Jedoch sind auch andere konstruktive Lösungen möglich.

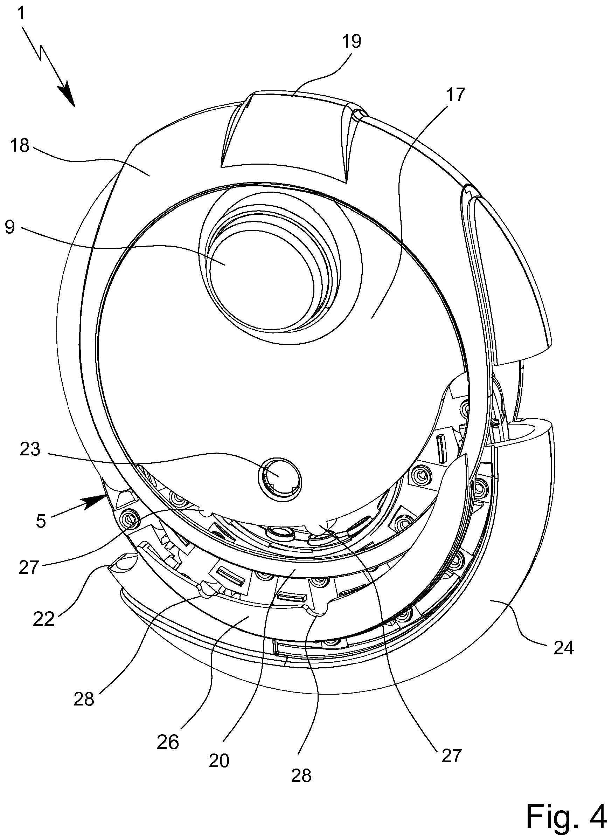

Fig. 4 zeigt sehr schematisch und nur ausschnittsweise den Inhalator 1 kurz vor dem vollständigen Ansetzen oder Einsetzen des Gehäuseteils 24.

Funktional ist das Gehäuseteil 24 bezüglich der in dem zugeordneten Magazin 15 enthaltenen Formulierung 2 vorzugsweise kodiert. Dies ist insbesondere dann bevorzugt, wenn das Gehäuseteil 24 mit dem Magazin 5 mit verschiedenen Formulierungen 2 zum Austausch bzw. Nachfüllen oder generell zusammen mit einem Inhalator 1 angeboten wird, um ein Verwechseln der Formulierungen 2 auszuschließen und damit die Bediensicherheit zu erhöhen.

Die Kodierung kann beispielsweise dadurch erreicht werden, dass das Gehäuseteil 24 mindestens ein erstes Kodierelement 27, beim Darstellungsbeispiel zwei oder mehr Kodierelemente 27, und der Inhalator bzw. dessen Gehäuse 17 auch mindestens ein zweites Kodierelement 28, beim Darstellungsbeispiel zwei oder mehr Kodierelemente 28 aufweist. Die Kodierung ergibt sich beispielsweise durch jeweilige Form, Größe und/oder Lage, insbesondere in Umfangsrichtung, der Kodierelemente 27, 28. Bei entsprechender bzw. fassender Kodierung sind die ersten und zweiten Kodierelemente 27, 28 insbesondere komplementär zueinander ausgebildet. Beispielsweise kann es sich um Vorsprünge und Vertiefungen bzw. Aussparungen handeln. Die Kodierung kann beispielsweise auch nur durch Variation der Umfangslage der Kodierelemente 27, 28 festgelegt werden. Nur bei passender Kodierung der ersten Kodierelemente 27 einerseits und der zweiten Kodierelemente 28 andererseits ist das dem Gehäuseteil 24 zugeordnete Magazin 5 mit dem Inhalator 1 verwendbar bzw. in diesem vollständig einsetzbar.

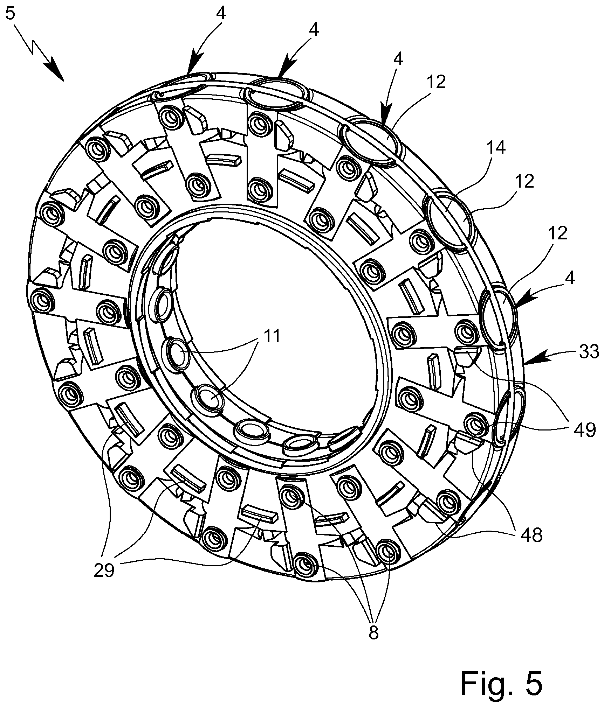

Fig. 5 zeigt das vorschlagsgemäße Magazin 5 gemäß einer ersten Ausführungsform, welches insbesondere bei dem Inhalator 1 gemäß der ersten Ausführungsform eingesetzt werden kann. In der perspektivischen Ansicht gemäß Fig. 5 ist ersichtlich, weist das Magazin 5 vorzugsweise axiale Haltemittel, insbesondere axiale Vorsprünge 29, auf, die besonders bevorzugt als sich in Umfangsrichtung erstreckende Stege oder dergleichen ausgebildet sind. Diese dienen insbesondere der bevorzugten unlösbaren Verbindung des Magazins 5 mit dem zugeordneten Gehäuseteil 24.

Das Magazin 5 ist vorzugsweise zumindest im Wesentlichen ringförmig bzw. ringscheibenförmig ausgebildet.

Das Magazin 5 weist vorzugsweise eine Vielzahl von Kapselkammern 4 mit darin aufgenommenen bzw. eingesetzten Kapseln 3 auf, wobei die Kapseln 3 in Fig. 5 nicht erkennbar sind.

Die Kapseln 3 und/oder Kapselkammern 4 sind mit ihren Längsachsen zumindest im Wesentlichen radial ausgerichtet.

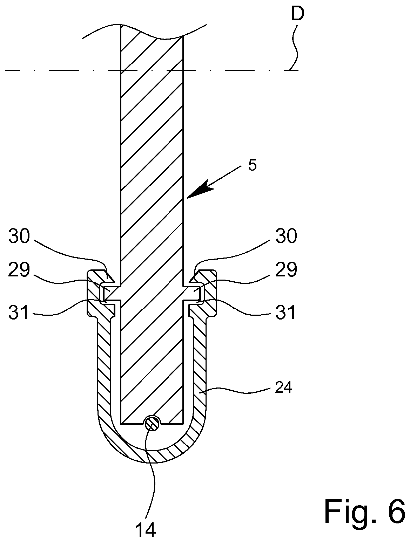

Fig. 6 zeigt in einem schematischen Teilschnitt in radialer Richtung, wie das Gehäuseteil 24 mit dem zugeordneten Magazin 5 unlösbar aber relativ zueinander drehbar verbunden sein kann. Das Gehäuseteil 24 weist vorzugsweise Halteabschnitte 30 auf, die die, vorzugsweise auf beiden Seiten des Magazins 5 gebildeten Vorsprünge 29 radial hintergreifen, so dass ein radiales Trennen des Gehäuseteils 24 vom Magazin 5 nicht möglich ist.

Besonders bevorzugt können die Vorsprünge 29 in entsprechend auf beiden Seiten im Gehäuseteil 24 gebildeten Ringnutabschnitten 31 eingreifen bzw. geführt sein, um eine gewünschte Drehlagerung des Magazins 5 zu erreichen. Jedoch kann dies beispielsweise auch dadurch erreicht werden, dass das Magazin 5 mit seiner Umfangsfläche oder seinem umfangsseitig angeordneten Sicherungselement 14 an einer Innenseite des Gehäuseteils 24 gleitend widergelagert ist. Weiter sind auch sonstige konstruktive Lösungen möglich.

Die Halteabschnitte 30 sind vorzugsweise hakenartig oder rastnasenförmig ausgebildet oder mit einer Einführschräge oder dergleichen versehen, insbesondere um zu ermöglichen, dass das Magazin 5 durch radiales Einschieben in das Gehäuseteil 24 mit diesem rastend verbindbar ist. Jedoch sind auch andere konstruktive Lösungen möglich.

In Fig. 3 ist angedeutet, dass das Gehäuseteil 24 - vorzugsweise auf entgegengesetzten Seiten - vorzugsweise eine entsprechende Vertiefung oder Durchbrechung 32 zum insbesondere axialen Eingriff des Gehäuses 17 oder der Abdeckung 18, insbesondere über den Ringabschnitt 20 oder Sicherungsabschnitt 25 oder dergleichen aufweist. Jedoch sind auch andere konstruktive Lösungen zum insbesondere rastenden und/oder unlösbaren bzw. belastbaren Verbindungen des Gehäuseteils 24 mit dem Inhalator 1 bzw. Gehäuse 17 möglich.

Vorzugsweise sind der Inhalator 1 und das Magazin 5 derart ausgebildet, dass das Magazin 5 nur in einer bestimmten Drehposition einsetzbar und/oder nur in einer bestimmten Drehposition entnehmbar bzw. wechselbar ist. Beispielsweise kann das Magazin 5 nur in einer Anfangsstellung, beispielsweise mit einer ersten Kapselkammer 4 benachbart zum Mundstück 15 in den Inhalator 1 bzw. dessen Gehäuse 17 einsetzbar sein. Vorzugsweise muss das Magazin 5 vollständig entleert sein, bevor es wieder entnehmbar bzw. wechselbar ist. Hierzu kann das Magazin 5 die gleiche Position wie beim Einsetzen erreicht haben oder sich beispielsweise in einer letzten oder Endposition befinden.

Vorzugsweise weist das Magazin 5 einen Ring- oder Halteabschnitt 55 auf, der beispielsweise als umlaufender Rand ausgebildet ist und axial vorsteht und beispielsweise mindestens einen oder zwei radiale Schlitze an gewünschten Drehpositionen aufweist, wie schematisch in Fig. 1 angedeutet. Der Inhalator 1 bzw. dessen Gehäuse 17 weist vorzugsweise einen Sicherungsabschnitt 56 auf, der axial vorsteht und in das Magazin 5 bzw. dem vom Halteabschnitt 55 gebildeten Rand derart eingreift, dass das Magazin 5 vorzugsweise nur in einer vorbestimmten Drehposition in den Inhalator 1 bzw. dessen Gehäuse 17 einsetzbar und/oder diesem entnehmbar ist. Jedoch sind auch andere konstruktive Lösungen möglich.

Fig. 5 zeigt einen bevorzugten Aufbau des Magazins 15 gemäß der ersten Ausführungsform. Dieses weist vorzugsweise einen Träger 33 zur Aufnahme der separat bzw. getrennt ausgebildeten oder vorgefertigten Kapselkammern 4 auf.

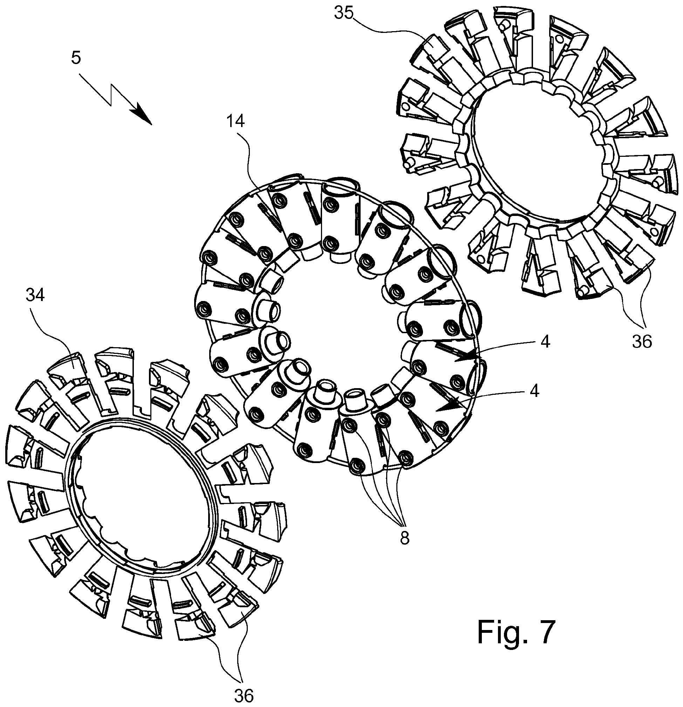

Beim Darstellungsbeispiel ist der Träger 33 vorzugsweise mehrteilig ausgebildet und insbesondere aus zwei Trägerelementen 34 und 35 aufgebaut, wie in der explosionsartigen Ansicht gemäß Fig. 7 gezeigt. Die Kapselkammern 4 sind axial zwischen den beiden Trägerelemente 34 und 35 einsetzbar bzw. zwischen diesen halterbar.

Insbesondere sind hierzu entsprechende radiale Haltearme 36 von dem Träger 33 bzw. den Trägerelementen 34 und 35 gebildet, die die Kapselkammern 4 im zusammengebauten Zustand formschlüssig halten.

Die beiden Trägerelemente 34 und 35 sind vorzugsweise rastend miteinander verbindbar. Jedoch sind auch andere konstruktive Lösungen möglich.

Der Träger 33 ist vorzugsweise derart ausgebildet, dass er die Kapselkammern 4 in einer definierten Ausrichtung bzw. Drehlage formschlüssig festlegt bzw. hält.

Das Sicherungselement 14 sichert die Kapselkammern 4 am bzw. im Träger 33.

Das Sicherungselement 14 ist vorzugsweise ringförmig bzw. spannringartig ausgebildet.

Das Sicherungselement 14 ist vorzugsweise einstückig ausgebildet.

Das Sicherungselement 14 ist vorzugsweise drahtförmig ausgebildet und/oder besteht vorzugsweise aus Metall, insbesondere Draht.

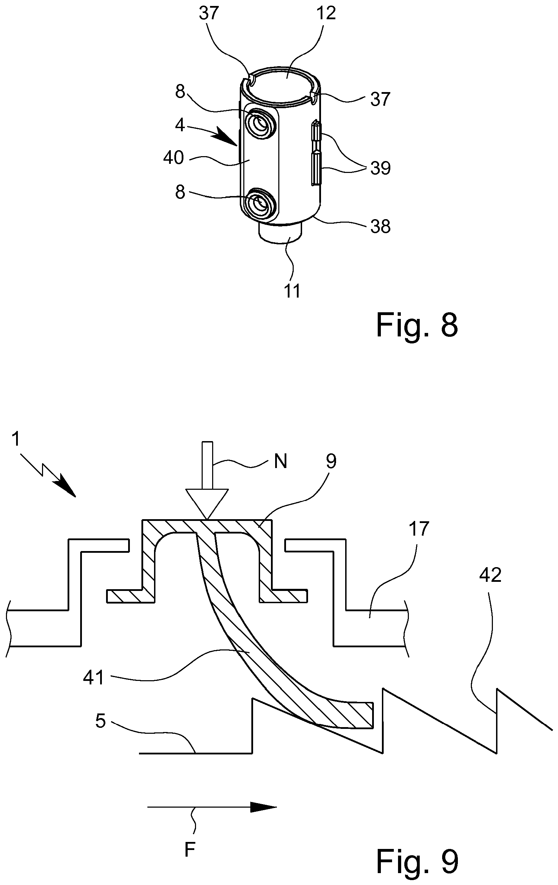

Die Kapselkammern 4 werden vorzugsweise vorgefertigt bzw. als separate Teile hergestellt. Fig. 8 zeigt in einer perspektivischen Ansicht schematisch eine bevorzugte Gestaltung der Kapselkammer 4.

Die Kapselkammer 4 ist vorzugsweise zumindest im Wesentlichen zylindrisch ausgebildet. Dieses ist insbesondere durch den vorzugsweise zumindest im Wesentlichen zylindrischen Aufnahmebereich für die zugeordnete Kapsel 3 (nicht dargestellt) im Inneren der Kapselkammer 4 vorteilhaft. Jedoch kann die Kapselkammer 4 grundsätzlich jede sonstige äußere Form aufweisen.

Die Kapselkammer 4 weist vorzugsweise eine Eingriffsmöglichkeit, insbesondere eine Kerbe 37, beim Darstellungsbeispiel zwei Kerben 37, auf, in die das Sicherungselement 14 eingreifen kann. Die Eingriffsmöglichkeiten bzw. Kerben 37 dienen insbesondere zur axialen Halterung bzw. Festlegung des Sicherungselements 14. Alternativ oder zusätzlich können die Eingriffsmöglichkeiten bzw. Kerben 37 auch der Festlegung der Kapselkammern 4 in ihrer Drehlage dienen, da bei jeder Kapselkammer 4 vorzugsweise gegenüberliegend zwei Kerben 37 abgebildet sind.

Die Eingriffsmöglichkeiten bzw. Kerben 37 sind vorzugsweise auslassseitig bzw. am äußeren axialen Ende, insbesondere in der axialen Stirnfläche, der Kapselkammern 4 gebildet. Anstelle von Kerben oder Vertiefungen können die Eingriffsmöglichkeiten auch durch Vorsprünge oder dergleichen gebildet sein.

Die radiale Lage der Kapselkammer 4 kann insbesondere durch radiale Anlage am inneren Ende, insbesondere im Bereich des Einlasses 11 oder einer äußeren Ringschulter 38, festgelegt werden.

Alternativ oder zusätzlich kann die Kapselkammer 4 jeweils auch mindestens ein Eingriffselement 39 aufweisen, um die Kapselkammer 4 in ihrer Drehlage und/oder in ihrer Radiallage im Magazin 5 definiert und insbesondere formschlüssig festlegen bzw. halten zu können. Beim Darstellungsbeispiel ist das Eingriffselement 39 beispielsweise als Vorsprung und/oder stegartig ausgebildet und/oder seitlich an der Kapselkammer 4 angeordnet bzw. angeformt. Weiter ist es auch möglich, dass beispielsweise zwei Eingriffselemente 39 auf entgegengesetzten Seiten angeordnet sind. Es sind auch sonstige Gestaltungen und Anordnungen des Eingriffselements 39 möglich.

Die Anstech- bzw. Nadelöffnungen 8 sind in der Kapselkammer 4 vorzugsweise bereits vorgeformt oder beispielsweise als dünnwandige Durchbrechungsstellen ausgebildet. Bedarfsweise können die Nadelöffnungen 8 also wahlweise offen oder öffenbar ausgebildet sein. Weiterhin ist es auch möglich, dass die Nadelöffnungen 8 selbstschließend ausgebildet sind, beispielsweise in der Art eines Septums, einer Membran oder dergleichen.

Im Darstellungsbeispiel sind die Nadelöffnungen 8 insbesondere durch ein anderes Material, beispielsweise ein besser anstechbares oder durchdringbares oder weicheres oder besser dichtendes oder selbstschließendes Material als das Grundmaterial, aus dem die Kapselkammer 4, hier zumindest im Wesentlichen hergestellt bzw. aufgebaut ist, gebildet. Das Grundmaterial ist hingegen vorzugsweise relativ starr bzw. hart.

Beim Darstellungsbeispiel sind die Nadelöffnungen 8 vorzugsweise jeweils in einem Materialbereich 40 aus dem anderen Material gebildet, wie Fig. 8 angedeutet. Hierbei kann für jede Nadelöffnung 8 ein gesonderter Materialbereich 40 oder für beide Nadelöffnungen 8 zusammen ein gemeinsamer Materialbereich 40, wie beim Darstellungsbeispiel angedeutet, vorgesehen sein.

Der die ein- oder zwei Nadelöffnungen 8 bildende Materialbereich 40 wird insbesondere durch Zwei-Komponenten-Spritzguss oder dergleichen aus dem anderen Material hergestellt.

Das Anstechen der Kapselkammern 4 erfolgt vorzugsweise von einer Flachseite des Magazins 5 bzw. axial oder zumindest im Wesentlichen axial also zumindest im Wesentlichen parallel zu der Drehachse D des Magazins 5. Die Nadelöffnungen 8 sind daher vorzugsweise an einer Flachseite (bedarfsweise auch an gegenüberliegenden Flachseiten) des Magazins 5 bzw. seitlich an den Kapselkammern 4 angeordnet.

Zur Benutzung öffnet ein nicht dargestellter Benutzer bzw. Patient den Inhalator 1 durch Schwenken bzw. Öffnen der Abdeckung 18 in die Schwenkrichtung S (Fig. 2). Hierdurch wird das Mundstück 15 freigegeben. Weiterhin erfolgt dann eine Betätigung der Öffnungseinrichtung 6 insbesondere durch Eindrücken des Betätigungselements 9. Hierdurch wird die sich in der Ausgangsposition A, also benachbart zum Mundstück 15 befindende Kapsel 3 in ihrer Kapselkammer 4 geöffnet bzw. mittels der Nadeln 7 angestochen. Nach Loslassen des Betätigungselements 9 werden die Nadeln 7 wieder zurückgezogen und die geöffnete Kapsel 3 kann sich frei in ihrer Kapselkammer 4 bewegen.

Anschließend erfolgt die Inhalation. Der Benutzer bzw. Patient nimmt das Mundstück 15 in den Mund und saugt Luft ein. Die Luft wird über den Einlass 11 angesaugt und strömt durch die Kapselkammer 4 bzw. den Aufnahmeabschnitt für die Kapsel 3 weiter über den Auslass 12 durch das Mundstück 15. Dieser Luftstrom bewirkt, dass sich die Kapsel 3 insbesondere in Richtung des Luftstroms hin- und herbewegt bzw. vibriert oder schwingt und dadurch den Austrag der Formulierung 2 aus der geöffneten Kapsel 3 und ein feines Dispergieren der Formulierung 2 im Luftstrom unterstützt. Es ist anzumerken, dass das Dispergieren vom Vibrationsweg, also insbesondere von der freien Bewegungslänge der Kapsel 3 innerhalb der Kapselkammer 4, abhängt bzw. beeinflusst wird. Beim Darstellungsbeispiel wird dieser Weg insbesondere von dem Sicherungselement 14 auslassseitig und von der inneren Ringschulter 13 einlassseitig begrenzt.

Nach der Inhalation, also nach der Entleerung der sich in der Ausgabeposition A befindlichen Kapsel 3 kann der Inhalator 1, also die Abdeckung 18, wieder geschlossen werden.

Für die nächste Inhalation ist es erforderlich, die nächste Kapsel 3 (und Kapselkammer 4) in die Ausgabeposition A, also die dem Mundstück 15 nächstliegende Position zu bewegen. Beim Darstellungsbeispiel genügt es, das Magazin 5 entsprechend weiterzubewegen bzw. weiterzudrehen. Hierfür bieten sich verschiedene Möglichkeiten an.

Die Bewegung bzw. Schwenkbewegung der Abdeckung 18 kann zum Drehen des Magazins 5 eingesetzt werden. Insbesondere kann die Öffnungs- oder Schließbewegung der Abdeckung 18 dazu verwendet werden, das Magazin 5 zur nächsten Kapsel 3 und Kapselkammer 4 weiter zu drehen. Dies kann beispielsweise über eine Mitnahmeklinke, Raste oder dergleichen an der Abdeckung 18, die in geeigneter Weise am Magazin 5 bzw. deren Träger 33 eingreift, erfolgen. Hierbei ist zu berücksichtigen, dass der Drehwinkel des Magazins 5 zur nächsten Kapsel 3 bzw. Kapselkammer 4 geringer sein kann bzw. ist, als die Schwenkbewegung der Abdeckung 18 beim Öffnen oder Schließen. Dementsprechend ist eine nur teilweise Kopplung der Bewegungen notwendig bzw. sinnvoll. Jedoch besteht grundsätzlich auch die Möglichkeit, das Öffnen oder Schließen der Abdeckung 18 derart anzupassen, insbesondere also eine von 90° verschiedene Schwenkbewegung und eine entsprechende Winkelanordnung der Kapseln 3 und Kapselkammern 4 im Magazin 5 vorzusehen, so dass bei jeweils vollständiger Mitnahme bzw. vollständigen Weiterdrehen des Magazins 5 über den gesamten Öffnungs- oder Schließwinkel der Abdeckung 18 nacheinander alle Kapseln 3 und Kapselkammern 4 in die Ausgabeposition A bewegt werden. In diesem Fall wird das Magazin 5 bis zur vollständigen Entleerung gegebenenfalls insgesamt sogar um mehr als eine Umdrehung gedreht. Die Kapseln 3 werden dann nicht in der Reihenfolge ihrer Anordnung im Magazin 5 entleert. Dementsprechend ist bei der vorliegenden Erfindung der Begriff "nächste Kapsel" grundsätzlich dahingehend zu verstehen, dass es sich hier nicht um die geometrisch nächste Kapsel 3 im Magazin 5 handeln muss (auch wenn dies bevorzugt ist), sondern dass es sich auch um die nächste zu entleerende Kapsel handeln kann.

Eine weitere Möglichkeit das Magazin 5 zur nächsten Kapsel 3 weiterzudrehen, besteht darin, einen Antrieb, wie einen Federantrieb, insbesondere mittels eines Federelements 52 (in Fig. 2 schematisch angedeutet), wie einer Uhren- oder Schenkelfeder oder dergleichen, vorzusehen. In diesem Fall erfolgt das Drehen des Magazins 5 durch diesen Antrieb. Es muss lediglich eine Freigabe zum Weiterdrehen des Magazins 5 zur nächsten Kapsel 3 erfolgen. Diese Freigabe kann beispielsweise durch Öffnen oder Schließen der Abdeckung 18, beispielsweise bei Erreichen einer bestimmten Position oder Überfahren einer bestimmten Zwischenposition, oder sonstige Betätigung, wie des Betätigungselements 9 der Öffnungseinrichtung 6, Bewegen des Mundstücks 15 oder dergleichen erfolgen.

Der Federantrieb bzw. das Federelement 52 kann beispielsweise so vorgespannt sein, dass es zum vollständigen Entleeren des Magazins 5, also zum Durchlaufen aller gewünschten Drehlagen, nicht neu gespannt werden muss. Alternativ kann das Federelement 52 auch durch das Bewegen der Abdeckung 18, insbesondere das Schwenken zum Öffnen und/oder Schließen der Abdeckung 18, (zusätzlich) gespannt werden.

Eine weitere, bevorzugte Möglichkeit, das Magazin 5 anzutreiben bzw. weiter zu drehen, besteht darin, die Bewegung bei der Betätigung des Betätigungselements 9 in eine entsprechende Antriebsbewegung für das Magazin 5 umzuwandeln. Fig. 9 zeigt in einem schematischen Schnitt eine konstruktive Möglichkeit, so dass bei Betätigung des Betätigungselements 9 das Magazin 5 zur nächsten Kapsel 3 weitergedreht bzw. weitergetaktet wird.

Vorzugsweise ist ein insbesondere zungenartiges Antriebselement 41 vorgesehen, dass mit dem Betätigungselement 9 insbesondere derart gekoppelt ist, dass die Niederdrückbewegung in eine Drehbewegung bzw. Querbewegung, also eine Förderbewegung F des Magazins 5, umgewandelt wird. Hierzu ist das Antriebselement 41 vorzugsweise flexibel ausgebildet oder angelenkt und greift beispielsweise mit seinem freien Ende an einer Zahnung 42 am Magazin 5 bzw. dessen Träger 33 an, so dass bei Wiederbetätigung der Öffnungseinrichtung 6 bzw. des Betätigungselements 9 - genauer gesagt insbesondere vor dem jeweiligen Anstechen einer sich in der Ausgabeposition A befindlichen Kapsel 3 - zunächst ein Weiterdrehen bzw. Weitertakten des Magazins 5 zur nächsten Kapsel 3 erfolgt. Jedoch sind hier auch andere konstruktive Lösungen möglich.

Der Inhalator 1 und das Magazin 5 sind vorzugsweise derart ausgebildet, dass das Magazin 5 jeweils definierte Drehstellungen einnimmt, in denen jeweils eine Kapselkammer 4 bzw. Kapsel 3 korrekt in der Ausgabeposition A positioniert ist, insbesondere so, dass sich der Verbindungsabschnitt 16 möglichst dicht an den Auslass 12 der in der Ausgabeposition A befindlichen Kapselkammer 4 anschließt und/oder so dass ein sicheres Anstechen, insbesondere genaues Einführen der Nadeln 7 in die Nadellöcher 8 ermöglicht wird. Hierzu sind insbesondere entsprechende Eingriffsmöglichkeiten am Magazin 5 bzw. dessen Träger 33, beispielsweise über die Vorsprünge 29, gebildet, die dann mit entsprechenden Rast-, Feder- oder Halteelementen am Gehäuse 17, Gehäuseteil 24 oder einer sonstigen Komponente des Inhalators 1 zusammenwirken.

Alternativ oder zusätzlich ist optional auch eine nicht dargestellte Abdichtung durch eine Dichtelement oder dergleichen zwischen der jeweiligen Kapselkammer 4 bzw. dessen Auslass 12 einerseits und dem sich anschließenden Mundstück 15 bzw. dessen Verbindungsabschnitt 16 andererseits möglich.

Nachfolgend werden weitere bevorzugte Ausführungsformen anhand der weiteren Figuren näher erläutert, wobei insbesondere nur wesentliche Unterschiede oder neue Aspekte näher beschrieben werden. Die bisherigen Ausführungen und Erläuterungen gelten daher insbesondere ergänzend oder entsprechend.

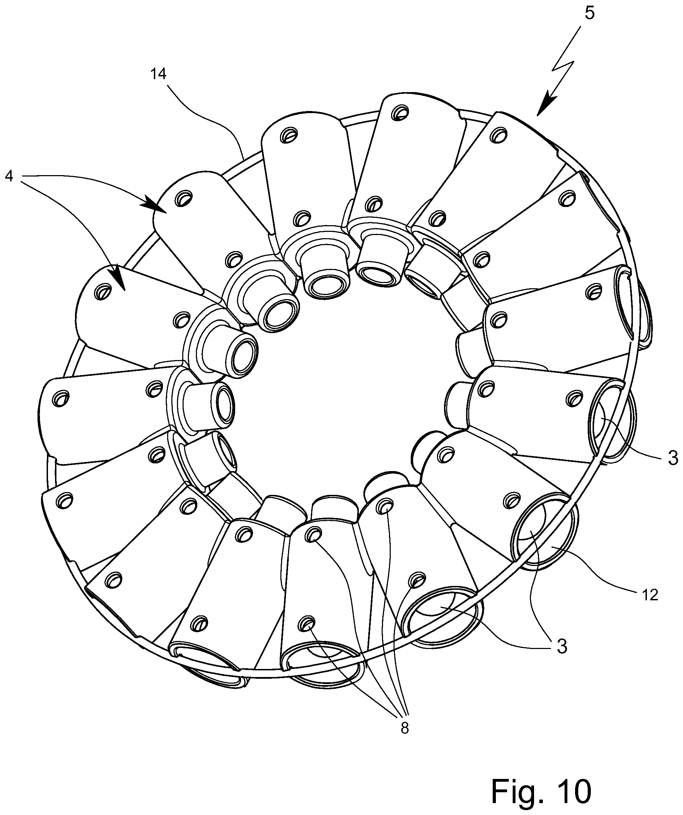

Fig. 10 zeigt in einer schematischen perspektivischen Ansicht eine zweite Ausführungsform des vorschlagsgemäßen Magazins 5. Hier sind die Kapselkammern 4 alle direkt miteinander verbunden, bilden also insbesondere eine einstückige oder zusammengebaute Einheit. Hier kann insbesondere ein Träger 33 entfallen, oder aber zur Stabilisierung vorgesehen sein.

Vorzugsweise sind die Kapseln 3 bei dieser Ausführungsform von außen in die Kapselkammern 4 eingebracht bzw. eingesetzt und dann - hier durch das Sicherungselement 14 - gesichert.

Alternativ kann das Magazin 5 bzw. können die Kapselkammern 4 auch durch zwei Schalen oder Hälften oder dergleichen gebildet sein, die miteinander verbunden werden.

Vorzugsweise sind die Kapselkammern 4 hier aus einem gummiähnlichen Material, TPE oder dergleichen hergestellt, insbesondere derart, dass Kontaktstellen bzw. Verbindungen insbesondere ohne zusätzliche Mittel abgedichtet oder zumindest weitgehend dicht sind.

Wie bei der ersten Ausführungsform weist das Magazin 5 gemäß der zweiten Ausführungsform beim Darstellungsbeispiel vorzugsweise 15 Kapselkammern 4 auf und enthält dementsprechend 15 Kapseln 3. Um das Magazin 5 zur nächsten Kapsel 3 weiterzubewegen bzw. weiterzutakten, muss das Magazin 5 dementsprechend um 24° gedreht werden, genauso wie bei der ersten Ausführungsform.

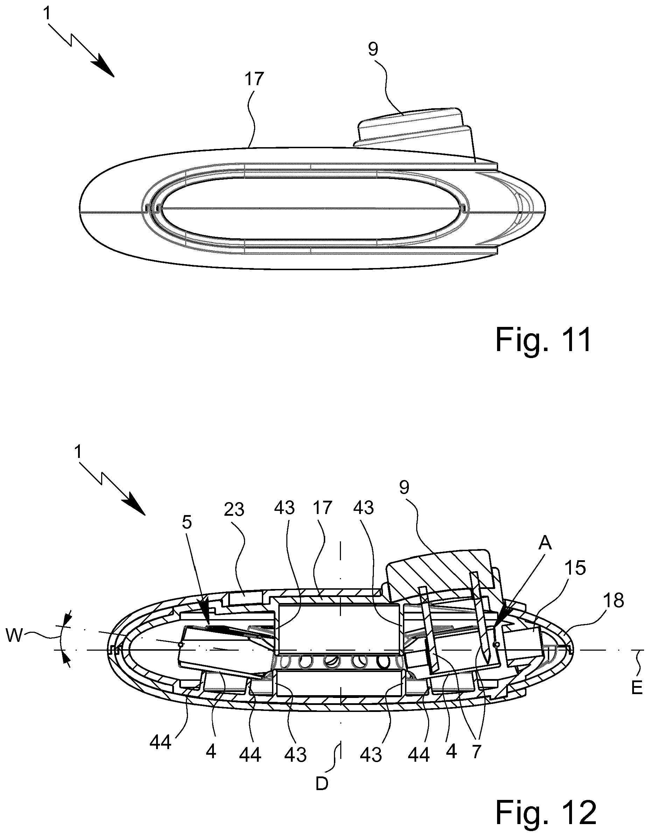

Fig. 11 zeigt in einer Seitenansicht eine zweite Ausführungsform des vorschlagsgemäßen Inhalators 1. Fig. 12 zeigt einen schematischen Schnitt des Inhalators 1, jedoch ohne Kapseln 3.

Hier sind die Kapselkammern 4 und Kapseln 3 mit ihren Längsachsen zwar grundsätzlich wieder zumindest im Wesentlichen radial bezüglich der Scheibenebene E des Magazins 5 bzw. flachen oder scheibenartigen Inhalators 1 oder bezüglich der Drehachse D des Magazins 5 ausgerichtet, jedoch etwas geneigt, insbesondere um einen Winkel W von etwa 5 bis 25° und insbesondere um etwa 10°. Diese Neigung erleichtert insbesondere die Befüllung der Kapselkammern 4 mit Kapseln 3.

Die Öffnungseinrichtung 6 bzw. die Nadeln 7 sind vorzugsweise dementsprechend geneigt, wie im Schnitt gemäß Fig. 12 angedeutet.

Die Öffnungseinrichtung 6 bzw. deren Betätigungselement 9 kann auch unabhängig von der bevorzugten Neigung der Kapselkammern 4 und Kapseln 3 um den Winkel W selbst entsprechend insbesondere zur Drehachse D des Magazins 5 bzw. zur Zentralachse des Inhalators 1 hin geneigt sein.

Die vorgenannten Gestaltung führt dazu, dass eine angenehme Bedienung und ein gutes Halten in der Hand eines nicht dargestellten Benutzers bzw. Patienten erleichtert oder unterstützt wird, insbesondere wenn der Inhalator 1 bzw. dessen Gehäuse 17 rückseitig entsprechend gewölbt oder seitlich geneigt ausgebildet wird.

Ein weiterer Vorteil der genannten Gestaltung liegt darin, dass ein besonders kompakter Aufbau bzw. geringer Durchmesser realisierbar ist, so dass der Inhalator 1 insbesondere sehr leicht und einfach in eine Tasche oder dergleichen aufgenommen werden kann.

Fig. 12 ist zu entnehmen, dass der Inhalator 1 bzw. dessen Gehäuse 17 vorzugsweise Lagerabschnitte 43, 44 zur drehbaren Lagerung des Magazins 5 im Inhalator 1 aufweist. Insbesondere dienen die Lagerabschnitte 43 einer radialen Lagerung und greifen vorzugsweise in die Mitte des Magazins 5 ein und/oder im Bereich der innenseitigen bzw. einlassseitigen Enden an den Kapselkammern 4 an.

Alternativ oder zusätzlich greifen die Lagerabschnitte 43 vorzugsweise an einer oder beiden axialen Seiten am Magazin 5 bzw. an den Kapselkammern 4 an.

Die Lagerabschnitte 44 dienen insbesondere einer axialen Lagerung und greifen vorzugsweise axial an den Kapselkammern 4 bzw. seitlich am Magazin 5 an.

Insbesondere erfolgt eine Widerlagerung des Magazins 5 bzw. der jeweiligen Kapselkammer 4 im Bereich der Ausgabeposition A bzw. auf der der Öffnungseinrichtung 6 abgewandten flachen Seite des Magazins 5, benachbart zur Öffnungseinrichtung 6, um eine Widerlagerung der jeweiligen Kapselkammer 4 beim Anstechen zu gewährleisten, hier durch mindestens einen Lagerabschnitt 44.

Die Lagerabschnitte 43 und/oder 44 sind vorzugsweise stegartig, rippenartig und/oder ringartig ausgebildet und/oder einstückig am Gehäuse 17 angeformt. Jedoch sind auch andere konstruktive Lösungen möglich.

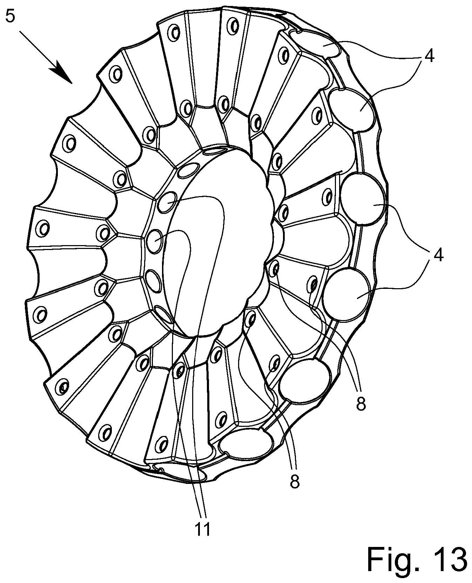

Fig. 13 zeigt in einer perspektivischen Ansicht eine dritte Ausführungsform des vorschlagsgemäßen Magazins 5. Diese Ausführungsform ist insbesondere bei dem in Fig. 11 und 12 dargestellten Inhalator 1 gemäß der zweiten Ausführungsform einsetzbar. Das Magazin 5 ist insbesondere als ein relativ weicher bzw. flexibler Ring ausgebildet, in dem die Kapselkammern 4 geformt oder in den die Kapselkammern 4 aufnehmbar oder einsteckbar sind. Besonders bevorzugt ist das Magazin 5 bzw. sind die Kapselkammern 4 hier aus einem elastischen bzw. relativ weichen und/oder gummiartigem Material hergestellt. Bei der Darstellung gemäß Fig. 13 sind die Kapseln 3 und das Sicherungselement 14 aus Vereinfachungsgründen weggelassen worden.

Alternativ (nicht gezeigt) kann das Magazin 5 statt der einstückigen Ausführung mit zusätzlichem Sicherungselement 14 auch aus zwei zueinander passenden Halbschalen oder Hälften (insbesondere mit einer Trennebene in etwa mittig längs durch die Kapselkammern 4) bestehen bzw. aufgebaut sein, insbesondere wobei die Kapseln 4 in die erste Halbschale eingesetzt werden und mit der zweiten Halbschale bedeckt werden.

Die Halbschalen sind dabei vorzugsweise rastend miteinander verbunden.

Vorzugsweise sind die Sicherungselemente 14 in Form von Stegen an den auslassseitigen Öffnungen der Kapselkammern 4 an den Halbschalen mit abgeformt.

Durch die vorzugsweise Verwendung von teilelastischem bzw. flexiblem Material für die Halbschalen liegen die Halbschalen an den Längsseiten der von ihnen gebildeten Kapselkammern dicht auf bzw. aneinander, so dass es zu keiner zusätzlichen Luftströmung durch die Längswände der Kapselkammern im Verbindungsbereich der Halbschalen kommt. Es ist jedoch auch denkbar, dass zusätzliche Dichtungen im Bereich der Verbindung der beiden Halbschalen eingesetzt werden.

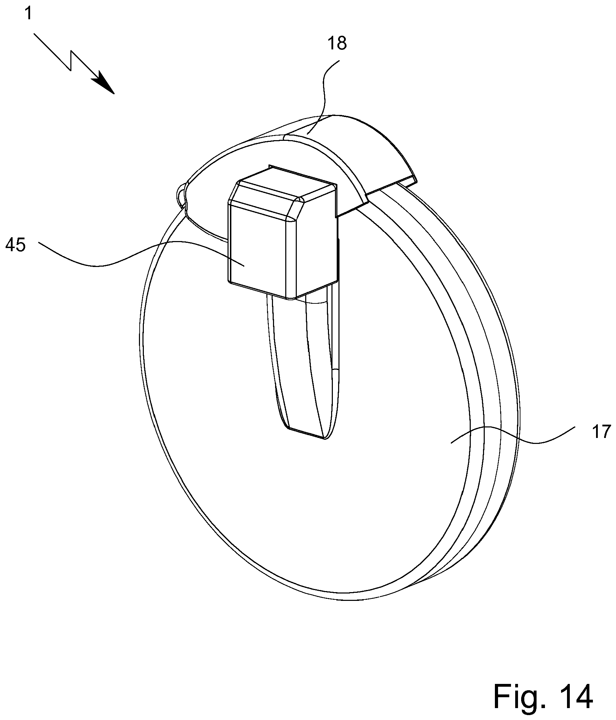

Fig. 14 zeigt in einer perspektivischen Ansicht eine dritte Ausführungsform des vorschlagsgemäßen Inhalators 1.

Bei der dritten Ausführungsform ist die Abdeckung 18 vorzugsweise peripher am Inhalator 1 angelenkt bzw. außen am Gehäuse 17 schwenkbar gelagert.

Bei der dritten Ausführungsform weist die Abdeckung 18 vorzugsweise einen Abdeckabschnitt 45 auf, der die Öffnungseinrichtung 6 bzw. deren Betätigungselement 9 bei geschlossener Abdeckung 18 abdeckt, so dass bei geschlossener Abdeckung 18 eine Betätigung des Betätigungselements 9 nicht möglich ist.

Das Magazin 5 ist hier in den Abbildungen zu dieser Ausführungsform vorzugsweise gemäß der ersten Magazin-Ausführungsform ausgebildet. Aspekte der anderen Magazin-Ausführungsformen sind jedoch auch hierauf übertragbar.

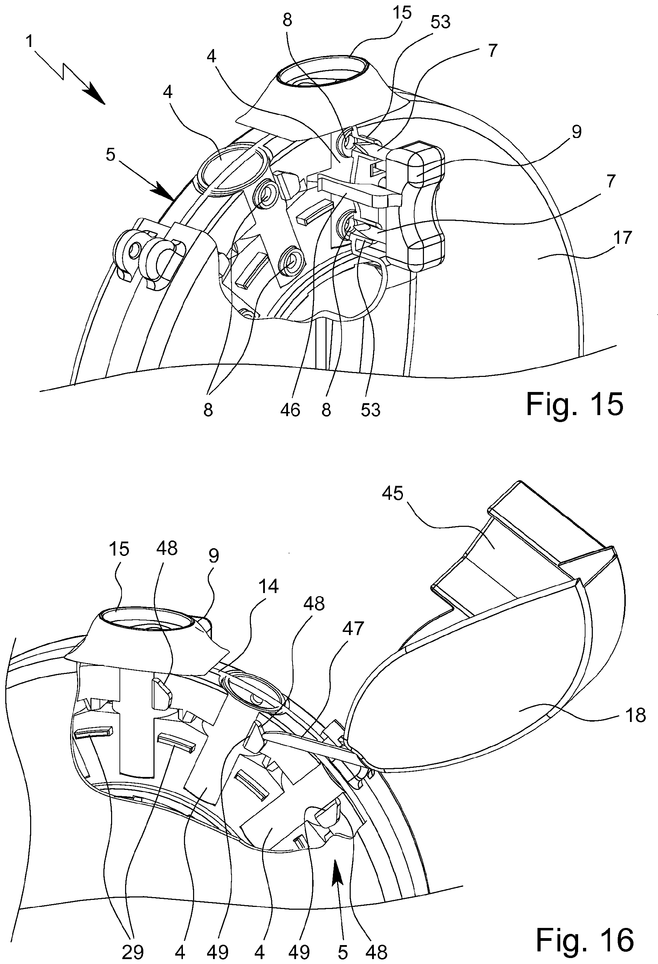

Fig. 15 zeigt in einer sehr schematischen Darstellung einen Ausschnitt des Inhalators 1 im Bereich des Mundstücks 15 und der Öffnungseinrichtung 6, wobei nur ein Teil des Magazins 5 dargestellt ist und das Gehäuse 17 und die Abdeckung 18 aus Veranschaulichungsgründen weggelassen sind. Die Öffnungseinrichtung 6 bzw. deren Betätigungselement 9 weist hier vorzugsweise eine Führung auf, die anstelle der bei der Erstausführungsform vorgesehenen hohlzylindrischen Führungshülse des Gehäuses 17 für das Betätigungselement 9 jetzt zusätzlich oder alternativ zwei vorzugsweise stegartige Führungselemente 46 aufweist, die im Gehäuse 17 geführt sind bzw. dieses durchgreifen und die beim Betätigen bzw. Eindrücken des Betätigungselements 9 auf beiden Seiten der in der Ausgabeposition A befindlichen Kapselkammer 4 in das Magazin 5 einfahren und dadurch die Kapselkammer 4 in der gewünschten Ausgabeposition A halten oder feinjustieren.

Allgemeiner ausgedrückt ist bei der dritten Ausführungsform des Inhalators 1 vorzugsweise die Öffnungseinrichtung 6 mit einer Justiereinrichtung versehen, um die jeweilige Kapselkammer 4 möglichst genau in die Ausgabeposition A zu bewegen und/oder dort zu halten, wenn das Öffnen bzw. Anstechen erfolgt. Hierzu erfolgt insbesondere ein entsprechender Eingriff in das Magazin 5, vorzugsweise in axialer Richtung, und/oder ein entsprechender Angriff an der jeweiligen Kapselkammer 4, beim Darstellungsbeispiel vorzugsweise mittels der Führungselemente 46.

Fig. 16 zeigt in einer schematischen Darstellung den Inhalator 1 im Bereich des Mundstücks 15 bei geöffneter Abdeckung 18, wobei das Gehäuse 17 aus Veranschaulichungsgründen weggelassen ist.

Bei der dritten Ausführungsform wird die Bewegung der Abdeckung 18 vorzugsweise zum Fördern bzw. Drehen bzw. Weitertakten des Magazins 5 eingesetzt. Insbesondere wird das Magazin 5 jeweils beim Öffnen der Abdeckung 18 zur nächsten Kapsel 3 bzw. Kapselkammer 4 weitergedreht.

Beim Darstellungsbeispiel weist der Inhalator 1 bzw. die Abdeckung 18 vorzugsweise ein insbesondere dorn- oder fingerartig ausgebildetes Antriebselement 47 auf, das an entsprechenden Angriffsabschnitten 48 am Magazin 5 bzw. Träger 33 angreifen kann.

Das Antriebselement 47 greift vorzugsweise zumindest im Wesentlichen von der Umfangsseite her durch eine entsprechende Ausnehmung im Gehäuse 17 in das Innere des Inhalators 1 ein.

Das Antriebselement 47 ist vorzugsweise federelastisch ausgebildet, so dass es insbesondere in Axialrichtung, also parallel zur Schwenkachse der Abdeckung 18 bzw. parallel zur Drehachse D des Magazins 5 ausfedern kann.

Die Angriffsabschnitte 48 bilden vorzugsweise axial vorspringende Schultern, an denen das Antriebselement 47 jeweils beim Öffnen der Abdeckung 18 zum Weiterdrehen des Magazins 5 in die nächste Kapselkammer 4 angreifen kann. Beim Schließen der Abdeckung 18 wird das Antriebselement 47 zurückbewegt und über eine schiefe Gleitfläche 49 axial ausgelenkt, so dass es über den nächsten Angriffsabschnitt 48 am Magazin 5 hinweggleiten kann, um erst bei der nächsten Öffnungsbewegung der Abdeckung 18 sich dann wieder in entgegengesetzter Richtung zu bewegen und dann an diesem Angriffsabschnitt 48 anzugreifen.

Das Magazin 5 ist vorzugsweise mittels einer Rücklaufsperre, Ratsche oder dergleichen gegen ein Zurückdrehen gesichert, so dass beim Schließen der Abdeckung 18 und Gleiten des Antriebselements 47 über die jeweilige Gleitfläche 49 kein unerwünschtes Zurückdrehen des Magazins 5 erfolgt.

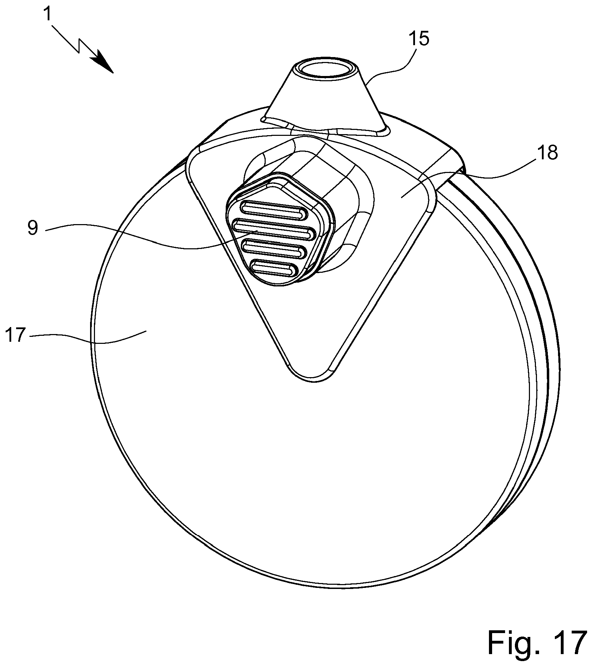

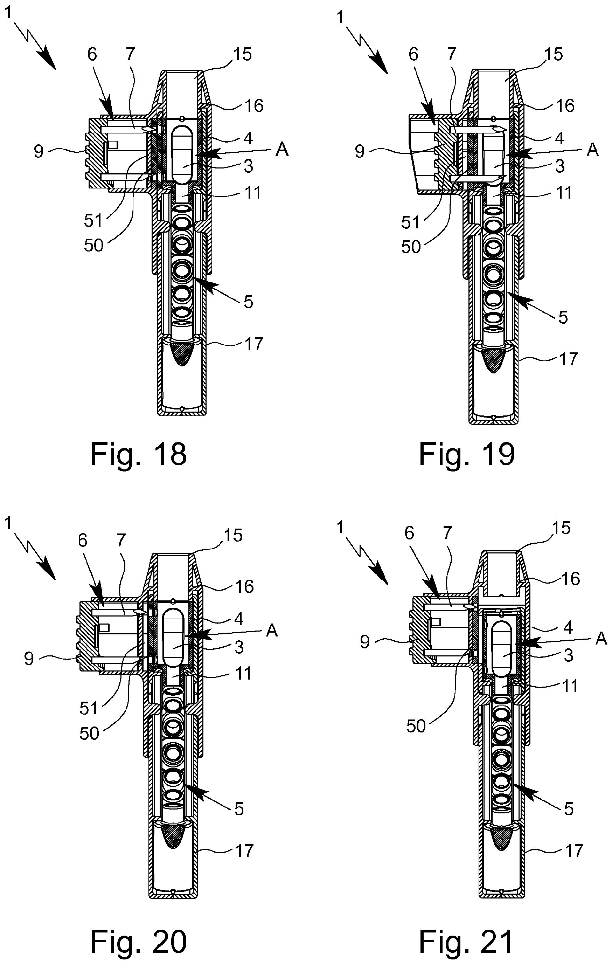

Fig. 17 zeigt in einer perspektivischen Ansicht eine vierte Ausführungsform des vorschlagsgemäßen Inhalators 1. Hier ist bzw. sind die Öffnungseinrichtung 6 und/oder das Mundstück 15, insbesondere beide zusammen, radial bewegbar, vorzugsweise um eine gute Abdichtung der Kapselkammer 4 bzw. Nadelöffnungen 8 nach dem Öffnen einer Kapsel 3 zu erreichen oder um das Magazin 5 weiter zur nächsten Kapselkammer 4 bzw. Kapsel 3 zu bewegen bzw. weiter zu takten. Die verschiedenen Funktionszustände und Funktionen werden nachfolgend anhand von den schematischen Vertikalschnitten gemäß Fig. 18 bis 21 näher erläutert.

Fig. 18 zeigt die Ausgangsposition. Die Kapsel 3 ist noch nicht angestochen. Eine Kapselkammer 4 mit der noch nicht angestochenen Kapsel 3 befindet sich in der Ausgabeposition A. Das Mundstück 15 ist nicht vertikal herausgezogen, sondern befindet sich in seiner radial eingeschobenen Position. Das Mundstück 15 bzw. dessen Verbindungsabschnitt 16 schließt sich also an die Kapselkammer 4 bzw. dessen Auslass 12 an.

Der Inhalator 1 weist vorzugsweise eine Verschlusseinrichtung zum Verschließen der Nadelöffnungen 8 - zumindest während der Inhalation - auf. Beim Darstellungsbeispiel weist die Verschlusseinrichtung ein außen an die Kapselkammer 4 anlegbares Dichtelement 50 auf oder ist durch dieses gebildet.

Das Dichtelement 50 ist insbesondere flach und/oder plattenartig oder rinnenartig gewölbt ausgebildet und/oder partiell an die Außenkontur der Kapselkammer 4 angepasst.

Das Dichtelement 50 ist von außen an die Kapselkammer 4 - insbesondere an die jeweilige Kapselkammer 4 bzw. nacheinander an die Kapselkammern 4 - anlegbar.

Insbesondere ist also ein gemeinsames Dichtelement 50 zum Verschließen der Nadelöffnungen 8 der jeweils in der Ausgabeposition A befindlichen Kapselkammer 4 bei der Inhalation vorgesehen.

Das Dichtelement 50 besteht vorzugsweise aus Kunststoff, insbesondere TPE (Thermoplastisches Elastomer) oder dergleichen.

Das Dichtelement 50 ist vorzugsweise derart ausgebildet, dass es selbstschließend und/oder selbst abdichtend ist. Selbstschließend kann insbesondere durch eine entsprechende Materialauswahl erreicht werden, so dass nach Durchstechen des Dichtelements 50 und Zurückziehen der Nadeln 7 sich die im Dichtelement 50 gebildeten Öffnungen wieder selbständig schließen. Hierzu kann das Dichtelement 50 auch membranartig oder filmartig ausgebildet sein.

Das Dichtelement 50 ist insbesondere der Öffnungseinrichtung 6 zugeordnet und/oder zusammen mit dieser und/oder zusammen mit dem Mundstück 15 relativ zu der Kapselkammer 4, dem Magazin 5 und/oder dem Gehäuse 17 und/oder in radialer Richtung bewegbar.

In der Ausgangsposition ist das Dichtelement 50 vorzugsweise von der sich in der Ausgabeposition A befindenden Kapselkammer 4 abgerückt, insbesondere in einer Vertiefung oder Ausnehmung 51 der Öffnungseinrichtung 6 aufgenommen, wie in Fig. 18 angedeutet.

Fig. 19 zeigt den Zustand nach der Betätigung. Das Betätigungselement 9 wurde eingedrückt. Die Nadeln 7 wurden vorwärts zur Kapselkammer 4 hin bewegt. Hierbei nehmen die Nadeln 7 das Dichtelement 50 zunächst mit und bringen es außen zur Anlage an der Kapselkammer 4. Weiter durchstechen bzw. durchdringen die Nadeln 7 das Dichtelement 50 (vollständig) und werden durch die Nadellöcher 8 hindurch in die Kapselkammer 4 hineinbewegt, so dass schließlich die darin befindliche Kapsel 3 angestochen wird, wie in Fig. 19 angedeutet.

Nach dem Öffnen bzw. Anstechen der Kapsel 3 wird das Betätigungselement 9 wieder losgelassen. Das Betätigungselement 9 und die Nadeln 7 ziehen sich selbsttätig, insbesondere aufgrund der Feder(n) 10 (in den Figuren 18-21 der Einfachheit halber nicht gezeigt, in ihrer Funktion jedoch wie in Fig.1 vorzugsweise vorhanden), wieder zurück, wobei das Dichtelement 50 jedoch in seiner abdichtenden und/oder an der Kapselkammer 4 anliegenden Lage bzw. Position verbleibt. Dieser Zustand ist in Fig. 20 angedeutet. In diesem Zustand dichtet das Dichtelement 50 die Nadellöcher 8 zumindest weitestgehend ab, um ein unerwünschtes Einströmen von Luft durch die Nadellöcher 8 beim Inhalieren zu vermeiden oder zumindest zu minimieren.

Vorzugsweise ist das Dichtelement 50 selbsthaftend ausgebildet und/oder wird eine derartige Materialpaarung für die Kapselkammer 4 bzw. deren Außenseite im Bereich der Nadelöffnungen 8 einerseits und für das Dichtelement 50 andererseits gewählt, dass eine relativ hohe Haftung erreicht wird, um zu erreichen oder sicherzustellen, dass das Dichtelement 50 auch bei Zurückziehen der Nadeln 7 an der Kapselkammer 4 haftet bzw. verbleibt, um die Nadelöffnungen 8 zumindest weitestgehend zu verschließen oder abzudecken.

Alternativ kann dem Dichtelement 50 auch eine Führung zugeordnet sein, entlang der das Dichtelement 50 durch einen kleinen Hebel z.B. am Betätigungselement 9 verschoben werden kann. Hierbei kann das Dichtelement 50 ebenfalls Öffnungen aufweisen, die durch ein Verschieben des Dichtelements 50 beim Betätigen des Betätigungselements 9, insbesondere beim Drücken des Betätigungselements 9, zur Deckung mit den Nadelöffnungen 8 der jeweiligen Kapselkammer 4 kommen, so dass die Nadeln 7 bei gedrücktem Betätigungselement 9 sowohl durch das Dichtelement 50 als auch durch die Wand der Kapselkammer 4 in den jeweils dazu vorgesehenen Öffnungen ragen. Werden die Nadeln 7 zurückgezogen bzw. zieht sich das Betätigungselement 9 zurück, wird das Dichtelement 50 mittels der Führung und/oder des Hebels oder dergleichen vorzugsweise verschoben, insbesondere so dass die Öffnungen des Dichtelements 50 nicht mehr deckungsgleich mit den Nadelöffnungen 8 in der Kapselkammer 4 sind bzw. dass das Dichtelement 50 die Nadelöffnungen 8 in der Kapselkammer 4 abdichtet bzw. abdeckt.

Der Inhalator 1 ist nun zur Inhalation bereit. Wenn die Inhalation erfolgt ist, wird das Mundstück 15 zusammen mit der Betätigungseinrichtung 9 radial nach außen bewegt bzw. gezogen. Dies erfolgt manuell durch einen nicht dargestellten Benutzer bzw. Patienten.

Fig. 21 zeigt den entsprechenden Zustand, wenn das Mundstück 15 radial abgerückt bzw. nach außen bewegt wurde.

Bei der Radialbewegung bzw. Relativbewegung wird das Dichtelement 50 mitgenommen und dadurch von der Kapselkammer 4 gelöst. Das Dichtelement 50 wird also (auch) relativ zu der Kapselkammer 4 bewegt, insbesondere in Richtung der Auflagefläche und/oder in Richtung der Längsachse der Kapselkammer 4. In dem abgerückten Zustand wird das Dichtelement 50 vorzugsweise wieder in der Ausnehmung 51 aufgenommen, wie in Fig. 21 angedeutet.

Schließlich wird das Mundstück 15 wieder radial eingeschoben bzw. zurückbewegt. Durch diese Bewegung wird vorzugsweise das Magazin 5 weiterbewegt bzw. weitergedreht, so dass die nächste Kapselkammer 4 in die Ausgabeposition A bewegt wird. Dieses Weiterbewegen bzw. Weiterdrehen kann durch eine entsprechende (betriebliche) Kopplung der Radialbewegung mit der Drehbewegung oder zur Umwandlung in eine Drehbewegung des Magazins 5 erfolgen. Alternativ kann beispielsweise auch eine Freigabe durch die Radialbewegung erfolgen, so dass das Magazin 5 beispielsweise mittels des Federelements 52 oder dergleichen zur nächsten Kapselkammer 4 weitergedreht werden kann. In diesem letztgenannten Fall erfolgt der Antrieb des Magazins 5 nicht durch die Radialbewegung bzw. Relativbewegung des Mundstücks 15, sondern durch die Schenkelfeder oder einen sonstigen Antrieb.

Wenn das Mundstück 15 wieder radial eingeschoben wurde, wird wieder die Ausgangsposition gemäß Fig. 18 eingenommen. Weiter wurde dann das Magazin 5 weiterbewegt bzw. weitergedreht; es befindet sich also eine neue Kapselkammer 4 mit einer noch ungeöffneten Kapsel 3 in der Ausgabeposition A. Der Inhalator 1 ist also zum Öffnen bzw. Anstechen der Kapsel 3 bereit.

Die verschiedenen Ausführungsformen des Inhalators 1 und einzelne Merkmale und Aspekte der verschiedenen Ausführungsformen können beliebig miteinander kombiniert, aber auch unabhängig realisiert werden. Gleiches gilt für das Magazin 5 und der Verwendung der verschiedenen Ausführungsformen des Magazins 5 in verschiedenen Inhalatoren 1.

Bevorzugt werden bei den hier beschriebenen Inhalatoren 1 und Magazinen 5 bzw. Kapseln 3 medizinische Formulierungen eingesetzt, die einen Bestandteil aufweisen, wie insbesondere in der WO 2009/115200 A1, vorzugsweise auf den Seiten 25 bis 40, oder in der EP 2 614 848 A1, vorzugsweise auf Seite 26, Zeile 21, bis Seite, 63 Zeile 2, genannt, oder dazu entsprechende Formulierungen aufweisen. Der Inhalt jener Zeilen wird hiermit vollumfänglich, auch zur Aufnahme von Merkmalen, in die vorliegende Anmeldung mit aufgenommen.

**Bezugszeichenliste:**

| | | | |
|---|---|---|---|
| 1 | Inhalator | 29 | Vorsprung |
| 2 | Formulierung | 30 | Halteabschnitt |
| 3 | Kapsel | 31 | Ringnutabschnitt |
| 4 | Kapselkammer | 32 | Vertiefung/Durchbrechung |
| 5 | Magazin | 33 | Träger |
| 6 | Öffnungseinrichtung | 34 | Trägerelement |
| 7 | Nadel | 35 | Trägerelement |
| 8 | Nadelöffnung | 36 | Haltearm |
| 9 | Betätigungselement | 37 | Kerbe |
| 10 | Feder | 38 | äußere Ringschulter |
| 11 | Einlass | 39 | Eingriffselement |
| 12 | Auslass | 40 | Materialbereich |
| 13 | innere Ringschulter | 41 | Antriebselement |
| 14 | Sicherungselement | 42 | Zahnung |
| 15 | Mundstück | 43 | Lagerabschnitt (radial) |
| 16 | Verbindungsabschnitt | 44 | Lagerabschnitt (axial) |
| 17 | Gehäuse | 45 | Abdeckabschnitt |
| 18 | Abdeckung | 46 | Führungselement |
| 19 | Ausbuchtung | 47 | Antriebselement |
| 20 | Ringabschnitt | 48 | Angriffsabschnitt |
| 21 | Anschlag | 49 | Gleitfläche |
| 22 | Anschlag | 50 | Dichtelement |
| 23 | Fenster | 51 | Ausnehmung |
| 24 | Gehäuseteil | 52 | Federelement |
| 25 | Sicherungsabschnitt | 53 | Nadelführung |
| 26 | Bogenabschnitt | 54 | Nadelführung |
| 27 | erstes Kodierelement (Gehäuse) | 55 | Halteabschnitt |
| 28 | zweites Kodierelement (Gehäuseteil) | 56 | Sicherungsabschnitt |
| A | Ausgabeposition | | |
| B | Bewegung | | |
| D | Drehachse | | |
| E | Ebene | | |
| H | Hauptausgaberichtung | | |
| S | Schwenkbewegung | | |
| N | Niederdrückbewegung | | |
| F | Förderbewegung | | |
| W | Winkel | | |

## Patentansprüche

1. Inhalator (1) zur Inhalation einer pulverförmigen Formulierung (2) aus einem Magazin (5), das ringförmig und um eine Drehachse drehbar gelagert ist und vordosierte Dosen der Formulierung (2) in separat voneinander öffenbaren Kapseln (3) enthält, wobei der Inhalator (1) ein zumindest im Wesentlichen flaches oder scheibenförmiges Gehäuse (17) mit einer Hauptebene (E) aufweist,wobei das Gehäuse (17) ein einsetzbares oder wechselbares Gehäuseteil (24) aufweist, das unlösbar mit dem drehbaren Magazin (5) gekoppelt ist, um ein Einsetzen bzw. Wechseln des Magazins (5) zu ermöglichen
**dadurch gekennzeichnet,**
**dass** das Magazin (5) in Richtung parallel zur Hauptebene des Magazins (5) in den Inhalator (1) einsetzbar ist.

2. Inhalator nach Anspruch 1, **dadurch gekennzeichnet, dass** das Gehäuseteil (24) einen Umfangsabschnitt entlang des Magazins (5) und/oder Gehäuses (17) bildet.

3. Inhalator nach einem der voranstehenden Ansprüche, **dadurch gekennzeichnet, dass** das Gehäuseteil (24) ein erstes Kodierelement (27) und das Gehäuse (17) ein zweites Kodierelement (28) aufweist, wobei die beiden Kodierelemente (27, 28) nur bei passender Kodierung ineinander eingreifen, so dass das dem Gehäuseteil (24) zugeordnete Magazin (5) nur bei passender Kodierung in den Inhalator (1) einsetzbar ist.

4. Inhalator nach einem der voranstehenden Ansprüche, **dadurch gekennzeichnet, dass** eine Abdeckung (18) eines Mundstücks (15) des Inhalators (1) derart mit dem Magazin (5) gekoppelt ist, dass ein Schwenken der Abdeckung (18) zum Öffnen oder Schließen des Mundstücks (15) das Magazin (5) insbesondere schrittweise dreht.

5. Inhalator nach einem der Ansprüche 1 bis 3, **dadurch gekennzeichnet, dass** ein Schwenken einer Abdeckung (18) eines Mundstücks (15) zum Öffnen oder Schließen des Mundstücks (15) ein Federelement (52) zum Weiterbewegen des Magazins (5) spannt.

6. Inhalator nach einem der voranstehenden Ansprüche, wobei das Magazin (5) eine Vielzahl von Kapseln (3) in Kapselkammern (4) aufweist, wobei die Kapseln (3) jeweils eine Dosis der Formulierung (2) enthalten und die Kapselkammern (4) ringförmig angeordnet sind, insbesondere wobei die Kapselkammern (4) als vorgefertigte Teile in das Magazin (5) eingesetzt sind, **dadurch gekennzeichnet, dass** die Kapseln (3) und/oder Kapselkammern (4) mittels eines gemeinsamen Sicherungselements (14) am bzw. im Magazin (5) derart gesichert sind, dass eine Abgabe der Formulierung (2) aus der jeweiligen Kapsel (3) ohne Entfernen des Sicherungselements (14) möglich ist, wobei das Sicherungselement (14) drahtförmig und/oder als Spannring ausgebildet ist.

7. Inhalator nach Anspruch 6, **dadurch gekennzeichnet, dass** das Magazin (5) einen einteiligen oder mehrteiligen Träger (33) zur Aufnahme der Kapselkammern (4) aufweist.

8. Inhalator nach Anspruch 7, **dadurch gekennzeichnet, dass** der Träger (33) flexibel bzw. gummielastisch ausgebildet ist.

9. Inhalator nach Anspruch 7, **dadurch gekennzeichnet, dass** der Träger (33) zumindest im Wesentlichen starr ausgebildet ist.

10. Inhalator nach einem der Ansprüche 6 bis 9, **dadurch gekennzeichnet, dass** das Sicherungselement (14) am äußeren Umfang des Magazins (5) angeordnet ist.

11. Inhalator nach einem der Ansprüche 6 bis 10, **dadurch gekennzeichnet, dass** die Kapseln (3) und/oder Kapselkammern (4) zumindest im Wesentlichen radial ausgerichtet sind.

12. Inhalator nach einem der Ansprüche 1 bis 5, wobei das Magazin (5) eine Vielzahl von Kapseln (3) in Kapselkammern (4) aufweist, wobei die Kapseln (3) jeweils eine Dosis der Formulierung (2) enthalten und die Kapselkammern (4) ringförmig angeordnet sind, **dadurch gekennzeichnet,**
**dass** die Kapseln (3) länglich ausgebildet und mit ihren Längsachsen schräg zur Ringebene (E) und/oder einer Drehachse (D) des Magazins (5) ausgerichtet sind.

13. Inhalator nach einem der Ansprüche 6 bis 12, **dadurch gekennzeichnet, dass** in den Kapselkammern (4) Anstechöffnungen vorgeformt oder als dünnwandige Durchbrechungsstellen ausgebildet sind.

14. Inhalator (1) nach einem der Ansprüche 1-5, wobei das Magazin (5) eine Vielzahl von Kapseln (3) in Kapselkammern (4) aufweist, wobei die Kapseln (3) jeweils eine Dosis der Formulierung (2) enthalten, und wobei der Inhalator (1) eine Öffnungseinrichtung (6) mit mindestens einer Nadel (7) zum Anstechen einer Kapsel (3) in einer Kapselkammer (4) durch mindestens eine Nadelöffnung (8) in der Kapselkammer (4) aufweist, **dadurch gekennzeichnet, dass** die Nadelöffnungen (8) selbstschließend ausgebildet sind.

15. Inhalator nach einem der Ansprüche 6 bis 14, **dadurch gekennzeichnet, dass** die Kapselkammer ein zumindest im Wesentlichen starres oder festes und/oder längliches Behältnis bzw. Kapselgehäuse mit einer insbesondere länglichen oder zylindrischen Kammer ist, in der die jeweilige Kapsel zur Entleerung in der Kapselkammer bewegbar bzw. in Vibration oder Schwingung versetzbar ist.

## Claims

1. Inhaler (1) for inhalation of a formulation (2) in powder form from a magazine (5) which is annular and rotatable around an axis of rotation and contains pre-dosed doses of the formulation (2) in capsules (3) which can be opened separately from one another, wherein the inhaler (1) has a housing (17) which is at least substantially flat or disc-shaped and has a main plane (E),
wherein the housing (17) has a housing part (24) which is insertable or replaceable and is undetachably coupled to the rotatable magazine (5) in order to facilitate an insertion or replacement of the magazine (5)
**characterised in that**
the magazine (5) can be inserted into the inhaler (1) in the direction parallel to the main plane of the magazine (5).

2. Inhaler according to claim 1, **characterised in that** the housing part (24) forms a circumferential portion along the magazine (5) and/or housing (17).

3. Inhaler according to any one of the preceding claims, **characterised in that** the housing part (24) has a first coding element (27) and the housing (17) has a second coding element (28), wherein the two coding elements (27, 28) only engage in one another when the coding matches, so that the magazine (5) associated with the housing part (24) can only be inserted into the inhaler (1) when the coding matches.

4. Inhaler according to any of the preceding claims, **characterised in that** a cover (18) of a mouthpiece (15) of the inhaler (1) is coupled to the magazine (5) in such a way that a pivoting of the cover (18) for opening or closing of the mouthpiece (15) rotates the magazine (5) in particular in steps.

5. Inhaler according to one of claims 1 to 3, **characterised in that** a pivoting of a cover (18) of a mouthpiece (15) for opening or closing of the mouthpiece (15)biases a spring element (52) for further movement of the magazine (5).

6. Inhaler according to any one of the preceding claims, wherein the magazine (5) has a plurality of capsules (3) in capsule chambers (4), wherein the capsules (3) in each case contain a dose of the formulation (2) and the capsule chambers (4) are arranged in an annular manner, in particular wherein the capsule chambers (4) are inserted as prefabricated parts into the magazine (5),
**characterised in that**
the capsules (3) and/or capsule chambers (4) are secured on or in the magazine (5) by means of a common securing element (14) in such a way that dispensing of the formulation (2) from the respective capsule (3) is possible without removal of the securing element (14), wherein the securing element (14) is in the form of wire and/or is configured as a clamping ring.

7. Inhaler according to claim 6, **characterised in that** the magazine (5) has an integral or multi-part support (33) to accommodate the capsule chambers (4).

8. Inhaler according to claim 7, **characterised in that** the support (33) is flexible or rubber-elastic.

9. Inhaler according to claim 7, **characterised in that** the support (33) is at least substantially rigid.

10. Inhaler according to any one of claims 6 to 9, **characterised in that** the securing element (14) is arranged on the outer circumference of the magazine (5).

11. Inhaler according to any one of claims 6 to 10, **characterised in that** the capsules (3) and/or capsule chambers (4) are oriented at least substantially radially.

12. Inhaler according to any one of claims 1 to 5, **characterised in that** the magazine (5) comprises a plurality of capsules (3) in capsule chambers (4), wherein the capsules (3) in each case contain a dose of the formulation (2) and the capsule chambers (4) are arranged in an annular manner, **characterized in**
**that** the capsules (3) are elongated and are oriented with their longitudinal axes obliquely with respect to the plane of the ring (E) and/or an axis of rotation (D) of the magazine (5).

13. Inhaler according to any one of claims 6 to 12, **characterised in that** piercing openings are pre-formed in the capsule chambers (4) or that thin-walled opening sites are formed in the capsule chambers.

14. Inhaler according to any one of claims 1 to 5, wherein the magazine (5) comprises a plurality of capsules (3) in capsule chambers (4), wherein the capsules (3) in each case contain a dose of the formulation (2), and wherein the inhaler (1) comprises an opening device (6) having at least one needle (7) for piercing a capsule (3) in a capsule chamber (4) through at least one needle opening (8) in the capsule chamber (4), **characterised in that** the needle openings (8) are self-closing.

15. Inhaler according to any one of claims 6 to 14, **characterised in that** the capsule chamber (4) is an at least substantially rigid or firm and/or elongated container or capsule housing having an in particular elongated or cylindrical chamber, in which the respective capsule is movable in the capsule chamber for emptying or can be made to vibrate or to oscillate.

## Revendications

1. Inhalateur (1) pour l'inhalation d'une préparation (2) pulvérulente à partir d'un magasin (5), qui est logé de manière annulaire et rotative autour d'un axe de rotation et contient des doses prédosées de la préparation (2) dans des capsules (3) ouvrables séparément les unes des autres, dans lequel l'inhalateur (1) présente un boîtier (17) au moins sensiblement plat ou en forme de disque avec un plan principal (E), dans lequel le boîtier (17) présente une partie de boîtier (24) insérable ou interchangeable qui est couplée de manière non détachable au magasin (5) rotatif afin de permettre une insertion ou un changement du magasin (5),
**caractérisé en ce que**
le magasin (5) peut être inséré en direction parallèle au plan principal du magasin (5) dans l'inhalateur (1).

2. Inhalateur selon la revendication 1, **caractérisé en ce que** la partie de boîtier (24) forme une section périphérique le long du magasin (5) et/ou du boîtier (17).

3. Inhalateur selon l'une quelconque des revendications précédentes, **caractérisé en ce que** la partie de boîtier (24) présente un premier élément de codage (27) et le boîtier (17) un deuxième élément de codage (28), dans lequel les deux éléments de codage (27, 28) viennent en prise seulement en cas de codage adapté l'un dans l'autre de sorte que le magasin (5) associé à la partie de boîtier (24) soit insérable seulement en cas de codage adapté dans l'inhalateur (1).

4. Inhalateur selon l'une quelconque des revendications précédentes, **caractérisé en ce qu'**un recouvrement (18) d'un embout (15) de l'inhalateur (1) est couplé au magasin (5) de telle manière qu'un pivotement du recouvrement (18) pour l'ouverture ou la fermeture de l'embout (15) tourne en particulier progressivement le magasin (15).

5. Inhalateur selon l'une quelconque des revendications 1 à 3, **caractérisé en ce qu'**un pivotement d'un recouvrement (18) d'un embout (15) pour l'ouverture ou la fermeture de l'embout (15) tend un élément de ressort (52) pour la poursuite du déplacement du magasin (5).

6. Inhalateur selon l'une quelconque des revendications précédentes, dans lequel le magasin (5) présente une pluralité de capsules (3) dans des chambres de capsule (4), dans lequel les capsules (3) contiennent chacune une dose de préparation (2) et les chambres de capsule (4) sont agencées en anneau, en particulier dans lequel les chambres de capsule (4) sont insérées comme parties préfabriquées dans le magasin (5), **caractérisé en ce que** les capsules (3) et/ou chambres de capsule (4) sont bloquées au moyen d'un élément de blocage (14) commun au niveau ou dans le magasin (5) de telle manière qu'une sortie de la préparation (2) de la capsule (3) respective soit possible sans retrait de l'élément de blocage (14), dans lequel l'élément de blocage (14) est réalisé en forme de fil et/ou comme anneau de serrage.

7. Inhalateur selon la revendication 6, **caractérisé en ce que** le magasin (5) présente un support (33) en une partie ou plusieurs parties pour la réception des chambres de capsule (4).

8. Inhalateur selon la revendication 7, **caractérisé en ce que** le support (33) est réalisé de manière flexible ou en caoutchouc élastique.

9. Inhalateur selon la revendication 7, **caractérisé en ce que** le support (33) est réalisé au moins sensiblement de manière rigide.

10. Inhalateur selon l'une quelconque des revendications 6 à 9, **caractérisé en ce que** l'élément de blocage (14) est agencé au niveau de la périphérie extérieure du magasin (5).

11. Inhalateur selon l'une quelconque des revendications 6 à 10, **caractérisé en ce que** les capsules (3) et/ou chambres de capsule (4) sont orientées au moins sensiblement radialement.

12. Inhalateur selon l'une quelconque des revendications 1 à 5, dans lequel le magasin (5) présente une pluralité de capsules (3) dans des chambres de capsule (4), dans lequel les capsules (3) contiennent respectivement une dose de la préparation (2) et les chambres de capsule (4) sont agencées en anneau, **caractérisé en ce**
**que** les capsules (3) sont réalisées de manière oblongue et sont orientées avec leurs axes longitudinaux en biais du plan annulaire (E) et/ou d'un axe de rotation (D) du magasin (5).

13. Inhalateur selon l'une quelconque des revendications 6 à 12, **caractérisé en ce que** dans des chambres de capsule (4), des ouvertures de perçage sont préformées ou sont réalisées comme points d'interruption à paroi mince.

14. Inhalateur (1) selon l'une quelconque des revendications 1 à 5, dans lequel le magasin (5) présente une pluralité de capsules (3) dans des chambres de capsule (4), dans lequel les capsules (3) contiennent respectivement une dose de la préparation (2), et dans lequel l'inhalateur (1) présente un dispositif d'ouverture (6) avec au moins une aiguille (7) pour le perçage d'une capsule (3) dans une chambre de capsule (4) par au moins une ouverture d'aiguille (8) dans la chambre de capsule (4), **caractérisé en ce que** les ouvertures d'aiguille (8) sont réalisées à fermeture automatique.

15. Inhalateur selon l'une quelconque des revendications 6 à 14, **caractérisé en ce que** la chambre de capsule est un récipient ou boîtier de capsule au moins sensiblement rigide ou solide et/ou oblong avec une chambre en particulier oblongue ou cylindrique, dans laquelle la capsule respective est mobile pour le vidage dans la chambre de capsule ou peut être amenée en vibration ou oscillation.
